# EUROPEAN PATENT APPLICATION

(11) **EP 3 782 647 A1**
(43) Date of publication of application: **24.02.2021**
(21) Application number: 18915773.8
(22) Date of filing: 27.11.2018
(51) Int. Cl.: A61K 45/00, A61K 31/7088, A61K 31/7105, A61K 31/713, A61K 48/00, A61P 1/16, A61P 9/00, A61P 11/00, A61P 13/12, A61P 17/00, A61P 43/00, C12Q 1/02, C12Q 1/6851, C12Q 1/686, G01N 33/15, G01N 33/50, G01N 33/53, G01N 33/68, C12N 15/113

(54) **ANTIFIBROTIC AGENT AND BIOMARKER FOR FIBROSIS**

(30) Priority: 17.04.2018 JP 2018079093
(71) Applicant: Osaka University, Suita-shi, Osaka 565-0871 (JP)
(72) Inventor: SATOH, Takashi, Suita-shi, Osaka 565-0871 (JP); AKIRA, Shizuo, Suita-shi, Osaka 565-0871 (JP)
(74) Representative: Hoffmann Eitle
(86) International application number: PCT/JP2018/043644
(87) International publication number: WO 2019/202767

(57) **Abstract**

The purpose of the present invention is to provide a molecule having a significant effect on the development of fibrosis and a substance capable of inhibiting fibrosis. This biomarker for fibrosis comprises RNA binding motif protein 7 (RBM7) and is useful as a biomarker for diagnosis of fibrosis. This fibrosis detection method comprising the step of detecting RBM7 in a biological sample from a subject preferably using an anti-RBM7 antibody is useful for diagnosis of fibrosis. This antifibrotic agent containing a substance which inhibits the expression of RBM7, preferably at least one nucleic acid drug selected from the group consisting of siRNAs, shRNAs, dsRNAs, miRNAs, and antisense nucleic acids against RBM7, is useful for treatment of fibrosis.

SEQUENCE LISTING

<110>  Osaka University

<120>  Biomarker of Fibrosis

<130>  18081WO

<150>  JP2018-079093
<151>  2017-4-17

<160>  2

<170>  PatentIn version 3.5

<210>  1
<211>  266
<212>  PRT
<213>  human

<400>  1

Met Gly Ala Ala Ala Ala Glu Ala Asp Arg Thr Leu Phe Val Gly Asn
1               5                   10                  15

Leu Glu Thr Lys Val Thr Glu Glu Leu Leu Phe Glu Leu Phe His Gln
            20                  25                  30

Ala Gly Pro Val Ile Lys Val Lys Ile Pro Lys Asp Lys Asp Gly Lys
        35                  40                  45

Pro Lys Gln Phe Ala Phe Val Asn Phe Lys His Glu Val Ser Val Pro
    50                  55                  60

Tyr Ala Met Asn Leu Leu Asn Gly Ile Lys Leu Tyr Gly Arg Pro Ile
65                  70                  75                  80

Lys Ile Gln Phe Arg Ser Gly Ser Ser His Ala Pro Gln Asp Val Ser
                85                  90                  95

Leu Ser Tyr Pro Gln His His Val Gly Asn Ser Ser Pro Thr Ser Thr
            100                 105                 110

Ser Pro Ser Arg Tyr Glu Arg Thr Met Asp Asn Met Thr Ser Ser Ala
        115                 120                 125

Gln Ile Ile Gln Arg Ser Phe Ser Ser Pro Glu Asn Phe Gln Arg Gln
    130                 135                 140

Ala Val Met Asn Ser Ala Leu Arg Gln Met Ser Tyr Gly Gly Lys Phe
145                 150                 155                 160

Gly Ser Ser Pro Leu Asp Gln Ser Gly Phe Ser Pro Ser Val Gln Ser

165                 170                 175

His Ser His Ser Phe Asn Gln Ser Ser Ser Ser Gln Trp Arg Gln Gly
            180                 185                 190

Thr Pro Ser Ser Gln Arg Lys Val Arg Met Asn Ser Tyr Pro Tyr Leu
        195                 200                 205

Ala Asp Arg His Tyr Ser Arg Glu Gln Arg Tyr Thr Asp His Gly Ser
    210                 215                 220

Asp His His Tyr Arg Gly Lys Arg Asp Asp Phe Phe Tyr Glu Asp Arg
225                 230                 235                 240

Asn His Asp Asp Trp Ser His Asp Tyr Asp Asn Arg Arg Asp Ser Ser
                245                 250                 255

Arg Asp Gly Lys Trp Arg Ser Ser Arg His
            260                 265

<210>  2
<211>  1939
<212>  DNA
<213>  human

<400>  2
tagggagggg gcgacgctga gatgggggcg gcggcggcgg aagcggatcg cactctcttt      60

gtgggcaacc ttgaaacgaa agtgaccgag gagctccttt tcgagctttt ccaccaggct     120

gggccagtaa taaaggtgaa aattccaaaa gataaggatg gtaaaccaaa gcagtttgcg     180

tttgtgaatt tcaaacatga agtgtctgtt ccttatgcaa tgaatctact taatggaatc     240

aaactttatg gaaggcctat caaaattcaa tttagatcag gaagtagtca tgccccacaa     300

gatgtcagtt tgtcatatcc ccaacatcat gttggaaatt caagccctac ctccacatct     360

cctagcaggt acgaaaggac tatggataac atgacttcat cagcacagat aattcagaga     420

tctttctctt ctccagaaaa ttttcagaga caagcagtga tgaacagtgc tttgagacaa     480

atgtcatatg gtggaaaatt tggttcttca cctctggatc aatcaggatt ttcaccatca     540

gttcaatcac acagtcatag tttcaatcag tcttcaagct cccagtggcg ccaaggtaca     600

ccatcatcac agcgtaaagt cagaatgaat tcttatccct acctagcaga tagacattat     660

agccgggaac agcgttacac tgatcatggg tctgaccatc attacagagg aaagagagat     720

gatttcttct atgaagacag gaatcatgat gactggagcc atgactatga taacagaaga     780

gacagtagta gagatggaaa atggcgctca tctcgacact aacacatgtt aaaaggacat     840

tgtttttata gggtcatttt aggccctttg actaagttga tatggaaata ttttgttgaa     900

aaactgtaca gagcagcttt acaagttgtc acatttcttt ataaattttt ttaaagctac 960

agtttaatac aaaatgaatt gcggttttat tacattaata acctttcacc tcaggttttt 1020

atgaagagga aagggtttta tgcaaaagaa agtgctacaa ttcctaatca ttttagacac 1080

tttaggaggg ggtgaagttg tatgataaag cagatatttt aattatttgt tatctttttg 1140

tattgcaaga aatttcttgc tagtgaatca agaaaacatc caggttgaca gtctaaaatg 1200

gctactggta ttttagttaa ttcaaaaatg aaacttttca gtgattcact ttactaacat 1260

tctatttgag aaggcttatt ggtaaagttt ggggataaag gcattgctta acttcttata 1320

taatttaggt ataaattctg tgacatgctc ttgagcttta ccctagttga acatacatgt 1380

gtagatttac acatactgtt tcattctaaa atttagaaat tgttcattaa atcccatttg 1440

aggtataagt cactcaggaa gttaaatatt ctctacacgt atatttttac attaaaaata 1500

cagtgttagc ataaatcccc ttttcaggaa gaacaaaaat gtcagtgcat agttagataa 1560

aatggtaaaa tgttttactg aaagcatact tttttggaaa atagattcat gaagccttta 1620

agtgctgctt ctgtcagtca aacgttaaaa actttaacat tttcaaagtg cccagactgt 1680

gtacaaagac acatgtaatg gagattgtac aggttgtttt tttgtttgaa cctttgaaag 1740

agtttaatct taacgttttc taattttaaa attttaaaat cttgtttaac aaaagcttgt 1800

attaagatac tgttttcatt tcattacaga attgtttata aaagttcatt tgttgaaaaa 1860

taaggatcct ttttaatacc acagcatttg tactgttcct ttttaatata ctgaaaaaat 1920

aaaaggaaaa aaaaaaaaa 1939

## Description

### TECHNICAL FIELD

The present invention relates to an antifibrotic agent and a biomarker of fibrosis. More specifically, the present invention relates to an antifibrotic agent, a biomarker of fibrosis, a method for detecting fibrosis, a diagnostic agent for fibrosis, and a method for screening an active ingredient of an antifibrotic agent.

### BACKGROUND ART

Fibrosis is a life-threatening disease caused by excessive formation of connective tissue and affects important organs (Non-Patent Documents 1 to 3). Then, it is known that innate immune cells play an important role in various intractable diseases caused by abnormal healing after inflammation or tissue damage, including fibrosis (Non-Patent Documents 4 to 7). It has been reported so far that several hematopoietic cell types are involved in fibrosis (Non-Patent Documents 8 to 11). Among them, SatM (Segregated nucleus containing Atypical Monocyte) and its regulatory factor C/EBPβ have been reported as immune system cells involved in onset of fibrosis (Non-Patent Document 11).

### PRIOR ART DOCUMENTS

### NON-PATENT DOCUMENTS

Non-Patent Document 1: J. Clin. Invest. 115, 209-218 (2005)
Non-Patent Document 2: Exp. Biol. Med. (Maywood) 233, 109-122 (2008)
Non-Patent Document 3: J. Hepatol. 56, 965-972 (2012)
Non-Patent Document 4: Immunity 34, 85-95 (2011)
Non-Patent Document 5: Cell 157, 832-844 (2014)
Non-Patent Document 6: Nature 495, 524-528 (2013)
Non-Patent Document 7: Nat. Immunol. 11, 936-944 (2010)
Non-Patent Document 8: Annu. Rev. Pathol. 8, 241-276 (2013)
Non-Patent Document 9: Nat. Rev. Immunol. 14, 181-194 (2014)
Non-Patent Document 10: Nature 496, 445-455 (2013)
Non-Patent Document 11: Nature volume 541, 96-101 (2017)

### SUMMARY OF THE INVENTION

### PROBLEMS TO BE SOLVED BY THE INVENTION

Full pathogenesis of fibrosis has not been clarified, and an effective drug has not yet been developed. In addition, although SatM is known to be important in initiation of fibrosis, a mechanism by which it migrates to a fibrotic area during fibrosis has not been elucidated.

The present inventors have focused on the fact that some molecules secreted by lung tissue at a fibrotic stage are involved in migration of SatM into an affected area, and a cell migration assay was performed by using a lung extract during a fibrotic phase. As a result, the present inventors have found that SatM was significantly recruited with an extract from wild-type (WT) lung after intratracheal administration of bleomycin (BLM) in the fibrotic phase rather than an inflammatory phase. This means that non-hematopoietic cells in the fibrotic phase produce an attractant, which causes SatM to migrate to the affected area and progress pulmonary fibrosis. Therefore, the present invention focuses on non-hematopoietic cells, and an object thereof is to provide a molecule that is important for an onset of fibrosis and a substance that can inhibit fibrosis.

### MEANS FOR SOLVING THE PROBLEM

The present inventors have conducted extensive studies and investigated mRNA and protein expression in the lungs of WT mice in a fibrotic phase after intratracheal administration of BLM, and found that among differentially expressed mRNA and proteins, RNA-binding motif protein 7 (RBM7) was significantly increased after BLM treatment. Of further note, the present inventors have found that in lung sections of patients with pulmonary fibrosis, expression of RBM7 was significantly elevated in areas of progressive fibrosis as compared to non-fibrotic control. RBM7 is known as a component of a nuclear exosome targeting complex that regulates degradation of various types of RNA, but little is known about its physiological or pathological role other than RNA processing function, and nothing is known about association of RBM7 with fibrosis. Furthermore, the present inventors have also found that fibrosis can be inhibited by suppressing the expression of RBM7 in vivo. The present invention has been accomplished by further studies based on this knowledge.

That is, the present invention provides inventions of the following aspects.
Item 1. An antifibrotic agent, containing a substance that suppresses expression of RNA-binding motif protein 7 (RBM7) as an active ingredient.
Item 2. The antifibrotic agent according to item 1, wherein the substance is at least one nucleic acid medicine selected from the group consisting of siRNA, shRNA, dsRNA, miRNA and an antisense nucleic acid against RBM7.
Item 3. The antifibrotic agent according to item 1 or 2, which is used for treatment, prevention or progression prevention of fibrosis.
Item 4. The antifibrotic agent according to item 3, wherein the fibrosis is fibrosis expressing RBM7.
Item 5. A biomarker of fibrosis, containing RBM7.
Item 6. The biomarker according to item 5, wherein the fibrosis is selected from the group consisting of pulmonary fibrosis, hepatic fibrosis, renal fibrosis, scleroderma, cardiac fibrosis, and myelofibrosis.
Item 7. A method for detecting fibrosis, including a step of performing a detection of RBM7 in a biological sample derived from a test subject.
Item 8. The method according to item 7, wherein the biological sample is a blood sample.
Item 9. The method according to item 7 or 8, wherein the detection is performed using an anti-RBM7 antibody.
Item 10. A diagnostic agent for fibrosis, containing an anti-RBM7 antibody.
Item 11. A method for screening an active ingredient of an antifibrotic agent, including the following steps (A) to (C):
   (A) bringing a test substance into contact with a cell expressing RBM7,
   (B) measuring an expression level of RBM7 in the cell, and
   (C) selecting the test substance as a candidate for an active ingredient of an antifibrotic agent when ability to suppress expression of RBM7 is recognized.
Item 12. A method for diagnosing fibrosis, including a step of performing a detection of RBM7 in a biological sample derived from a test subject, and a step of diagnosing fibrosis based on a detection result obtained in the above step.
Item 13.A method for treating, preventing or preventing progression of fibrosis, including a step of administering to a fibrosis patient a therapeutically effective amount of a substance that suppresses expression of RBM7.

### ADVANTAGES OF THE INVENTION

According to the present invention, RBM7 which is important for onset of fibrosis is provided as a biomarker. Since RBM7 is hardly expressed in normal cells, the method for detecting fibrosis of the present invention enables highly specific diagnosis of fibrosis.

Further, according to the screening method of the present invention, development of an excellent antifibrotic agent can be accelerated by using RBM7 as a target and screening a substance that suppresses expression of RBM7.

Furthermore, according to the antifibrotic agent of the present invention, fibrosis can be effectively inhibited by using RBM7 as a target and suppressing the expression of RBM7. In fact, in vivo fibrosis-inhibiting effect against fibrosis by siRNA against RBM7 has been confirmed.

### BRIEF DESCRIPTION OF THE DRAWINGS

Fig. 1a shows an evaluation of migration ability of SatM in lungs treated with PBS or BLM, which was performed by Diff Quick staining (n = 3). A scale bar shows 100 µm.
Fig. 1b shows an evaluation of migration ability of SatM in lungs treated with PBS or BLM, in which the migrated SatM was measured using luminescence as an index (n = 2).
Fig. 1c shows an evaluation of migration ability of hematopoietic cells (CD45+) and non-hematopoietic cells (CD45-) present in lung tissue, in which
   a lung on day 10 after PBS or BLM administration was divided into hematopoietic system and non-hematopoietic system, and
   SatM migration ability exhibited by those cells was measured using luminescence as an index (n = 4).
Fig. 1d shows Western blot (WB) analysis of non-hematopoietic cells of WT mouse lung tissue 10 days after PBS or BLM administration (n = 3).
Fig. 1e shows RBM7 expression in control and human fibrotic lungs (n = 9). A scale bar shows 100 µm.
Fig. 1f shows survival rates of BLM-treated WT or Rbm7-/- mice (n = 5).
Fig. 1g shows an evaluation of a fibrotic area by MRI of a mouse lung 10 days after BLM administration.
Fig. 1h shows Azan- (top), H&E- (middle) and αSMA stained (bottom) lung sections. A scale bar shows 100 µm.
Fig. 1i shows hydroxyproline content in mouse lungs 14 days after BLM administration.
Fig. 1j shows qPCR analysis of tgfb1 and collal mRNA in the mouse lungs (n = 3) 14 days after BLM administration.
Fig. 1k shows migration of SatM by FACS analysis of BAL (bronchoalveolar lavage) fluids (WT, n = 4; Rbm7-/-, n = 3) 10 days after BLM administration.
Fig. 1l shows, in models of hepatic fibrosis induced by choline-deficient-L-amino acid-high-fat diet (CDA-HFD, n = 3), images of livers after 12 weeks on normal diet (N.C.) or CDA-HFD, and H&E- (top) and Azan stained livers (bottom).
Fig. 1m shows hydroxyproline content in models of hepatic fibrosis induced by choline-deficient-L-amino acid-high-fat diet (CDA-HFD, n = 3).
Fig. In shows expression levels of RbM7 by WB analysis of livers from WT mice, in models of hepatic fibrosis induced by choline-deficient-L-amino acid-high-fat diet (CDA-HFD, n = 3).
Fig. 1o shows mRNA expression of tgfb1 and collal in models of hepatic fibrosis induced by choline-deficient-L-amino acid-high-fat diet (CDA-HFD, n = 3).
Fig. 1p shows, in models of renal fibrosis induced by unilateral ureteral obstruction (UUO, n = 3), H&E- (top), Azan- (middle) and Picrosirius Red (bottom) stained kidneys 7 days after UUO.
Fig. 1q shows hydroxyproline content in models of renal fibrosis induced by unilateral ureteral obstruction (UUO, n = 3).
Fig. 1r shows expression levels of RbM7 by WB analysis of kidneys from WT mice, in models of renal fibrosis induced by unilateral ureteral obstruction (UUO, n = 3).
Fig. Is shows mRNA expression of tgfb1 and collal in models of renal fibrosis induced by unilateral ureteral obstruction (UUO, n = 3).
Fig. 2a shows WB analysis of RBM7 expression on lung CD45 positive and negative cells in day 10 wild-type mice treated with BLM or PBS.
Fig. 2b shows immunohistostaining of RBM7 in wild-type mice treated with BLM or PBS.
Fig. 2c shows survival rates of BLM-treated bone marrow (BM) chimeric mice (n = 5). A p-value between § and † was p < 0.01.
Fig. 2d shows H&E (top) and Azan (bottom) staining obtained from BM chimera. A scale bar shows 100 µm.
Fig. 2e shows mRNA levels of tgfb1 and collal in lungs (n = 3) 14 days after BLM administration injection.
Fig. 2f shows hydroxyproline content in the lungs (n = 3) 14 days after BLM administration.
Fig. 2g shows WB analysis of lungs from WT mice after a lapse of predetermined days (d0, d1, d3, n = 3; d6, n = 4; d10, n = 3; d14, n = 4) after BLM administration.
Fig. 2h shows immunohistostaining of RBM7 expression in fibrotic human lungs (n = 9).
Fig. 2i shows WB analysis of lungs (n = 3/group) from WT mice after a lapse of predetermined days after BLM administration.
Fig. 2j shows survival rates of BLM-treated mice (mock, n = 23; Q-VD-OPh, n = 15) that received predetermined treatment from day 5 to day 8. A p-value between § and # was P < 0.01.
Fig. 2k shows H&E (top), Azan (middle) and TUNEL (bottom) staining of lungs 10 days after BLM administration. A scale bar shows 100 µm.
Fig. 2l shows the number of SatM cells in BAL (n = 3) 12 days after BLM inoculation.
Fig. 2m shows TUNEL staining of lung tissue of WT mice and Rbm7-/- mice after BLM ingestion (left), liver tissue of mice fed CDA-HFD for 12 weeks (middle), and kidney tissue 7 days after UUO (right).
Fig. 2n shows quantification of TUNEL-positive cells (n = 3 per group).
Fig. 2o is survival rates of mice after i.v. injection of lung-specific siRNA on BLM treatment and subsequent days 5 and 10 (fibrotic stage) (si control, n = 22; si-Rbm7 in fibrotic stage, n =18). A P value between # and † was P < 0.01.
Fig. 2p shows H&E (top) and Azan (bottom) staining of lungs 12 days after BLM treatment with predetermined siRNA treatment at the fibrotic stage (days 5 and 10). A scale bar shows 100 µm.
Fig. 2q shows H&E (top) and TUNEL (bottom) staining of lungs 10 days after BLM treatment administration. A scale bar shows 100 µm.
Fig. 2r is WB analysis of bone marrow (BM) chimeric mice day 10 after BLM administration.
Fig. 2s shows WB analysis of WT and Rbm7-/- HEK293 cells after treatment with 50 µM etoposide for 24 hours.
Fig. 2t shows WB analysis of WT or Rbm7-/- HEK293 cells transfected with control or RBM7 expression vector and treated with 50 µM etoposide for 24 hours.
Fig. 3a shows a scatter plot of apoptosis-related molecules in WT and Rbm7-/- HEK293 cells.
Fig. 3b shows a scatter plot of protein-coding genes for apoptosis-related molecules in WT and Rbm7-/- HEK293 cells.
Fig. 3c shows a heat map of IncRNA and antisense RNA expression in WT and Rbm7-/- HEK293 cells.
Fig. 3d shows a Venn diagram of ncRNAs detected by RNA-seq and RIP-seq of WT and Rbm7-/- HEK293 cells.
Fig. 3e shows a heat map of potential targets for Rbm7 from untreated WT and Rbm7-/- HEK293 cells.
Fig. 3f shows a heat map of potential targets for Rbm7 from etoposide-treated WT and Rbm7-/- HEK293 cells (50 µM, 6 hours).
Fig. 3g shows RNA- and RIP-seq data of NEAT1 and MALAT1 IncRNA.
Fig. 3h shows WB analysis of WT, Rbm7-/- or Rbm7-/- HEK293 cells in which designated ncRNA was knocked down treated with 50 µM etoposide for 24 hours.
Fig. 3i shows images of live cells (visible in green) and dead cells (visible in red) and their quantification in HEK293 cells by treatment with 50 µM etoposide for 24 hours.
Fig. 3j shows survival rates of BLM-treated mice (WT si-control, n = 17; Rbm7-/-si-control, n = 10; Rbm7-/-si-NEAT1, n = 10) with predetermined siRNA treatment on day 0 (6 hours before BLM administration), days 5 and 10. P values between § and # and between † and # were P < 0.01.
Fig. 3k shows H&E (top) and Azan (bottom) staining of lungs at 14 days after BLM administration with predetermined siRNA treatment (days 0, 5 and 10). A scale bar shows 100 µM.
Fig. 3l shows tgfb1 and collal mRNA levels in the lungs (WT si-control, n = 5; Rbm7-/- si-control, n = 5; Rbm7-/- si-NEAT1, n = 4) at 14 days after BLM treatment with predetermined siRNA treatment (days 0, 5 and 10).
Fig. 3m shows hydroxyproline content in the lungs (n = 4) at 14 days after BLM treatment with predetermined siRNA treatment (days 0, 5, and 10).
Fig. 4a shows qPCR analysis of NEAT1 and NEAT1.2 IncRNA in WT or Rbm7-/- HEK293 cells.
Fig. 4b shows qPCR analysis ofRNA expression levels of NEAT1 and NEAT1.2 in Rbm7-/- HEK293 cells transfected with a vector encoding empty full length Rbm7 or RNA-binding domain deletion mutant.
Fig. 4c shows images of NEAT1-containing (visible in red) paraspeckle in WT or Rbm7-/- HEK293 cells.
Fig. 4d shows quantification of NEAT1-containing paraspeckle in WT or Rbm7-/- HEK293 cells.
Fig. 4e shows images of in situ hybridization staining of NEAT1 (visible in red) in WT and Rbm7-/- mouse lungs.
Fig. 4f shows qPCR analysis (n = 3) of NEAT1 expression level in lung epithelial cells.
Fig. 4g shows images of NEAT1 RNA-FISH (visible in red) and DAPI (visible in blue) of WT and Rbm7-/- mouse primary lung epithelial cells.
Fig. 4h shows quantification of NEAT 1-containing paraspeckle in the mouse primary lung epithelial cells.
Fig. 4i shows staining of γ-H2AX (visible in green) and DAPI (visible in blue) of WT, Rbm7-/- or NEAT1 knockdown Rbm7-/- HEK293 cells treated with 50 µM etoposide for 6 hours.
Fig. 4j shows WB analysis of WT, Rbm7-/- or NEAT1 knockdown Rbm7-/- HEK293 cells after treatment with 50 µM etoposide for 6 hours.
Fig. 4k shows formaldehyde-crosslinked RIP-qPCR analysis of NEAT1 enrichment in HEK293 cells after immunoprecipitation with a predetermined antibody.
Fig. 4l shows WT or Rbm7-/- HEK293 cells stained for BRCA1 (visible in green), NEAT1 RNA (visible in red) and DAPI (visible in blue).
Fig. 4m shows WB analysis of WT, Rbm7-/- or Brca1 knockdown Rbm7-/- HEK293 cells treated with 50 µM etoposide for 24 hours.

### EMBODIMENTS OF THE INVENTION

### 1. Biomarker

The biomarker of fibrosis of the present invention is characterized by containing RNA-binding motif protein 7 (RBM7). Hereinafter, the biomarker of the present invention will be described in detail.

In the present invention, fibrosis is a disease caused by excessive formation of connective tissues and may be fibrosis of any organ. In the present invention, the biomarker RBM7 of fibrosis has been found to cause excessive cell death in a fibrotic phase, and since cell death commonly functions in any organ, biomarker RBM7 can be applied to fibrosis of any organ. Specific examples of fibrosis include pulmonary fibrosis, hepatic fibrosis, renal fibrosis, scleroderma, cardiac fibrosis, myelofibrosis, and the like.

RBM7 is known as a component of nuclear exosome targeting complex that regulates RNA degradation, and its amino acid sequence and a base sequence of mRNA encoding the same are registered in public databases. Specifically, a human RBM7 amino acid sequence is registered as Accession No. Q9Y580 in Uniprot/Swissprot, and a base sequence of mRNA encoding human RBM7 is registered as Accession No. AF156098 in GenBank.

Regarding human RBM7, a specific amino acid sequence of a protein is shown in SEQ ID NO: 1, and a specific base sequence of mRNA is shown in SEQ ID NO: 2. In RBM7, an RNA-binding domain at positions 1 to 91 in SEQ ID NO: 1 play an important role in fibrosis.

RBM7, the biomarker of fibrosis of the present invention, may be human RBM7 shown in SEQ ID NO: 1, or a mutant thereof. That is, examples of RBM7, the biomarker of fibrosis of the present invention, include human RBM7 shown in SEQ ID NO: 1, a mutant of human RBM7, RBM7 of a non-human species, and a mutant of RBM7 of a non-human species.

More specifically, examples of RBM7, the biomarker of fibrosis of the present invention, include the following proteins (1) to (3), preferred include the protein (1).
(1) A protein consisting of an amino acid sequence shown in SEQ ID NO: 1
(2) a protein in which one or several amino acids in the amino acid sequence shown in SEQ ID NO: 1 consists of an amino acid sequence substituted, deleted, inserted, and/or added, having activity as RBM7
(3) a protein having a sequence identity with the amino acid sequence shown in SEQ ID NO: 1 of 80% or more and having activity as RBM7

In the protein of (2) above, "one or several amino acids... substituted, deleted, inserted, and/or added" is not particularly limited, but the number (preferably 10 or less, more preferably 9 or less, more preferably 8 or less, more preferably 7 or less, more preferably 6 or less, more preferably 5 or less, more preferably 4 or less, more preferably 3 or less, more preferably 1 or 2, and most preferably 1) of amino acids that can be substituted, deleted, inserted, and/or added, by a known method for producing a mutant protein such as site-directed mutagenesis, are intended to be substituted, deleted, inserted, and/or added.

Also, in the protein (3), the sequence identity with the amino acid sequence shown in SEQ ID NO: 1 may be 80% or more, but is preferably 85% or more, 90% or more, 95% or more, further preferably 98% or more, and particularly preferably 99% or more. Here, the sequence identity with the amino acid sequence shown in SEQ ID NO: 1 is a sequence identity calculated by comparison with the amino acid sequence shown in SEQ ID NO: 1. Further, the "sequence identity" shows an identity value of an amino acid sequence obtained by BLAST PACKAGE [sgi32 bit edition, Version 2.0.12; available from National Center for Biotechnology Information (NCBI)] bl2seq program (Tatiana A. Tatsusova, Thomas L. Madden, FEMS Microbiol. Lett., Vol. 174, p247-250, 1999). Parameters may be set to Gap insertion Cost value: 11 and Gap extension Cost value: 1.

Further, in the protein of (2) or (3) above, the activity as RBM7 may be measured by measuring RNA processing activity of an arbitrary protein according to a well-known method. When the protein has RNA processing activity as a result of measurement, the protein can be determined as a protein having activity as RBM7.

### 2. Method for detecting fibrosis

The present invention provides a method for detecting fibrosis. The detection method of the present invention is characterized by including a step of performing a detection of RBM7 in a biological sample derived from a test subject.

### 2-1. Detection subject

The detection subject sample of the detection method of the present invention is a biological sample, and the detection subject substance is the biomarker (that is, RBM7).

### 2-2. Test subject

Fibrosis analyzed by the detection method of the present invention corresponds to fibrosis affected by a test subject from which a biological sample to be analyzed is derived. The test subject may be any animal, and specific examples include experimental animals such as rodents such as mice, rats, hamsters and guinea pigs and rabbits; livestock such as pigs, cows, goats, horses and sheep; pets such as dogs and cats; and primates such as humans, monkeys, orangutans and chimpanzees. The test subject in the present invention is preferably a primate, and further preferably a human.

Further, the biological sample may be any sample collected from the test subject, and includes blood samples, tissue samples and cell samples. From the viewpoint of non-invasiveness, the biological sample is preferably a blood sample. Examples of the blood sample include whole blood, serum, plasma, and the like. From the viewpoint of ease of preparation and diagnostic accuracy, the blood sample is preferably serum. Collection and preparation of various blood samples can be performed according to known methods. For example, serum can be prepared by removing blood cells and blood coagulation factors such as fibrinogen (factor I), prothrombin (factor II), factor V, and factor VIII from the collected blood (whole blood), and can be obtained as a supernatant obtained after allowing blood to stand, or as a supernatant obtained by subjecting blood to centrifugation. The blood sample is diluted to an appropriate concentration as needed, and used.

In addition, from the viewpoint of diagnostic accuracy, the biological sample preferably includes tissue samples and cell samples. Examples of the tissue samples and cell samples include samples obtained by biopsy, surgical pathological diagnosis, or cytology. Specifically, the tissue samples and cell samples may be fresh tissues and cells, frozen tissues and cells, and histopathologically processed (for example, formalin-fixed or paraffin-embedded) tissues and cells, and the like. These samples may be used as sections or used as protein-soluble fractions prepared from tissues or cells.

### 2-3. Anti-RBM7 antibody

In the detection method of the present invention, RBM7 is detected by using an anti-RBM7 antibody and specifically binding to the biomarker (RBM7) in the biological sample.

The anti-RBM7 antibody used to detect the biomarker in the biological sample in the detection method of the present invention is not particularly limited as long as the biomarker (RBM7) present in the biological sample collected from the test subject can be specifically recognized as an antigen, but an anti-RBM7 antibody derived from an animal belonging to the same species as the test subject is preferable. For example, when the test subject is human, the anti-RBM7 antibody used for detecting the biomarker is preferably of human origin.

Further, the anti-RBM7 antibody used to detect the biomarker in a blood sample is not particularly limited as long as it is detectable, and it may be any isotype such as IgG (including IgG1, IgG2, IgG3, IgG4 or the like), IgD, IgE, IgA, sIgA, or IgM. IgG is exemplified as a preferred antibody isotype in the present invention. Furthermore, the anti-RBM7 antibody may have at least a complementarity determining region (CDR) for specifically recognizing and binding RBM7, and specifically, it may be a binding fragment of an antibody such as (ab')₂, Fab', Fab, Fv, sFv, dsFv, or sdAb). The anti-RBM7 antibody can be prepared by genetic engineering according to a conventional method.

### 2-4. Detection of biomarkers

The detection method of the present invention can be performed by detecting the presence or absence of the biomarker in the biological sample collected from the test subject. Also, in the detection method of the present invention, the biomarker may be detected by measuring an amount of the biomarker in the biological sample. By measuring the amount of the biomarker and comparing the measured value with a value of the biomarker in a normal control group and/or the test subject suffering from fibrosis, more accurate analysis can be performed. Therefore, the detection method of the present invention is preferably performed by a method of quantifying the biomarker in the biological sample.

Specific examples of the method of detecting the biomarker present in the biological sample using an anti-RBM7 antibody include a method of directly or indirectly detecting a specific binding between the anti-RBM7 antibody and the biomarker RBM7 by contacting the anti-RBM7 antibody with the biological sample. Specific examples of such a detection method include immunoassays such as ELISA, Western blotting, immunoprecipitation, radioimmunoassay (RIA), fluorescent immunoassay, and immunohistological staining. When detecting the biomarker by these immunoassays, it can be performed by binding a label such as enzyme label, chromogenic label, radiolabel or luminescent label to an antibody that binds specifically to the biomarker RBM7 or its secondary antibody, and detecting or measuring the label.

The conditions for carrying out the immunoassay are not particularly limited as long as the specific binding between the biomarker RBM7 and the anti-RBM7 antibody in the biological sample can be detected, and are set based on conventionally known conditions.

For example, when detecting the biomarker RBM7 of the present invention by ELISA, a biological sample collected from the test subject is added to each well of a multi-well plate to which the anti-RBM7 antibody is fixed, and the anti-RBM7 antibody in the well is reacted with the biomarker RBM7 in a blood sample. Then, detection and/or quantification of the biomarker in the biological sample can be performed by detecting and/or quantifying a reaction product obtained by adding a labeled antibody that specifically binds to RBM7 derived from the test subject to each well to react them and then adding an enzyme substrate. Here, the labeled antibody can be appropriately selected based on the animal of the test subject from which the biological sample is collected, and when the test subject is a human, examples thereof include a non-human labeled antibody that specifically binds to human RBM7 (for example, rabbit-derived anti-human RBM7 antibody) and the like.

Further, also for an enzyme used for labeling the labeled antibody, it can be appropriately selected and used from those usually used, and examples thereof include peroxidase, alkaline phosphatase, luciferase, esterase, glucose oxidase, β-D-galactosidase, β-D-glucuronidase, and the like. Moreover, the enzyme substrate may be appropriately selected from known substrates according to the type of enzyme, and for example, when the enzyme is peroxidase, 3,3',5,5'-tetramethylbenzidine (TMB) can be used as a substrate.

Detection and/or quantification of the reaction product generated by the reaction of the enzyme and the substrate can be performed by measuring an absorbance of the reaction product, for example, 3,3',5,5'-tetramethylbenzidine (TMB) is used as the enzyme substrate, it can be carried out by measuring the absorbance at 450 nm.

In the case of radioimmunoassay (RIA), an anti-RBM7 antibody is labeled with a radioisotope, reacted with a biomarker RBM7 in a biological sample to form an immune complex, and can be detected based on radioactivity released from the radioisotope.

In the case of fluorescent immunoassay, detection can be performed by immobilizing an anti-RBM7 antibody on a plate or the like, adding a biological sample thereto to react them, then further reacting a labeled antibody that specifically binds to RBM7 present in the biological sample of the test subject, and detecting fluorescent color development. The labeled antibody that specifically binds to RBM7 derived from the test subject is as described in the ELISA, and one labeled with a fluorescent dye is used. Examples of the fluorescent dye include FITC, PE, APC, Cy-3, Cy-5, and the like.

In the case of immunoprecipitation, detection can be performed by reacting an anti-RBM7 antibody with a biological sample to form an immune complex and precipitating it as an insoluble matter using an active adsorbent such as protein A or protein G. Furthermore, immunoprecipitation and Western blotting can be combined for detection. More specifically, an anti-RBM7 antibody connected to a tag such as FLAG is reacted with a biological sample, and an immune complex is formed when the biomarker RBM7 is present in the sample, thus it is precipitated by the active adsorbent. Then, the obtained precipitate is subjected to Western blotting. That is, the precipitate is separated and developed by SDS-PAGE, and transferred to a nitrocellulose membrane, PVDF membrane or the like, then an antigen-antibody reaction is carried out on a transfer membrane with an antibody against the tag, whereby it can be detected as a band when the biomarker RBM7 is present in the biological sample.

When detecting the biomarker RBM7 of the present invention by immunohistological staining, a pathological section of tissue or cell tissue is prepared from a tissue sample or a cell sample, and subjected to deparaffinization and/or activation treatment as needed, then the anti-RBM7 antibody is used to specifically bind to the biomarker RBM7 present on a surface of the pathological section. Then, location of the specifically bound biomarker RBM7 can be determined, by visualization (staining) using a secondary antibody of the anti-RBM7 antibody as a labeled antibody. Visualization (staining) is performed through a change in signal by an enzyme antibody method, a fluorescent antibody method, an autoradiography method, or the like. The expression level of the biomarker RBM7 can be quantified by calculating a score value reflecting the staining state and digitizing it. Here, the labeled antibody can be appropriately selected based on the animal of the test subject from which the biological sample is collected, and when the test subject is a human, examples thereof include a human labeled antibody against the anti-RBM7 antibody.

### 2-5. Determination of susceptibility to fibrosis

When the biomarker is detected in the biological sample collected from the test subject by the detection method of the present invention, it can be determined that the test subject has fibrosis.

In the test subject suffering from fibrosis, the amount of the biomarker of the present invention in the biological sample is significantly increased as compared to the normal control group. Therefore, when quantitatively detecting the biomarker of the present invention, fibrosis can be diagnosed by comparing the amount of biomarker in the blood sample of the normal control group with the amount of biomarker in the biological sample derived from the test subject.

In the present invention, a fact that the amount of biomarker contained in the biological sample is higher than that in the normal control group specifically includes a case where a 95th percentile value is set as a cutoff value in the normal control group, and the biomarker amount of the test subject is equal to or higher than the cutoff value. When the biomarker amount of the test subject is equal to or higher than the cutoff value, it can be determined that the test subject is likely to have fibrosis. Also, when the biomarker amount of the test subject is less than the cutoff value, it may be determined that the test subject is less likely to have fibrosis. Moreover, the higher the amount of the biomarker of the present invention in the biological sample, the more severe the condition of fibrosis can be predicted to be.

In addition, prognosis of the test subject can be predicted by detecting the biomarker of the present invention. For example, a therapeutic effect can be monitored by performing the detection method of the present invention after treatment. In this case, the treatment can be terminated at a time when the biomarker of the present invention is not detected or a time when the detected amount is less than the cutoff value. Also, a timing of starting or resuming treatment can be monitored by performing the detection method of the present invention during follow-up. In this case, the treatment can be started or resumed at a time when the biomarker of the present invention is detected or a time when the detected amount is equal to or higher than the cutoff value.

### 3. Diagnostic agent for fibrosis

The present invention provides a diagnostic agent for fibrosis, which contains an anti-RBM7 antibody. The diagnostic agent of the present invention can be used for carrying out the method for detecting a muscle disease described above.

The anti-RBM7 antibody used for the diagnostic agent of the present invention is as described above.

In the diagnostic agent of the present invention, the anti-RBM7 antibody may be provided in a state of being immobilized on an insolubilized carrier. A material of the insolubilized carrier is not particularly limited as long as it does not interfere with the detection of biomarker, and examples thereof include polystyrene, polyethylene, polypropylene, polyester, polyacrylonitrile, polyvinyl chloride, fluororesin, crosslinked dextran, paper, silicon, glass, metal, agarose, and the like. Moreover, these materials may be used in combination of two or more types. Shape of the insolubilized carrier may be any shape, for example, microplate, tray shape, spherical shape, fibrous shape, rod shape, plate shape, container shape, cell, test tube or the like.

Immobilization of the anti-RBM7 antibody on the insolubilized carrier can be performed according to a conventionally known method.

An amount of anti-RBM7 antibody immobilized on the insolubilized carrier is not particularly limited as long as it is an amount sufficient to specifically bind to RBM7 in the biological sample, but is, for example, such an amount that a concentration of the anti-RBM7 antibody in a solution used when immobilized on the insolubilized carrier is 1 to 10 µg/mL.

Further, the diagnostic agent of the present invention may contain a buffer solution, a stabilizer, a preservative and the like, in addition to the anti-RBM7 antibody, and may be formulated according to a conventionally known method. Furthermore, the diagnostic agent of the present invention may include a labeled antibody that may be required to carry out the detection of RBM7, a detection agent for a labeling substance, a lysing agent, a detergent, a reaction stop solution, a control sample, a test protocol, and the like. The test protocol includes information such as operations and procedures for carrying out the method for detecting fibrosis described above.

### 4. Method for screening active ingredient of antifibrotic agent

The screening method of the present invention is a method for screening an active ingredient of an antifibrotic agent, and includes the following steps (A) to (C).
(A) bringing a test substance into contact with a cell expressing RBM7,
(B) measuring an expression level of RBM7 in the cell, and
(C) selecting the test substance as a candidate substance for an active ingredient of an antifibrotic agent when the expression level of RBM7 is reduced as compared to a case of measurement in the absence of the test substance.

### 4-1. Test substance

In the screening method of the present invention, the test substance may be any substance that can be a candidate substance for the active ingredient of the antifibrotic agent, and its type is not particularly limited. For example, the test substance may be a natural substance such as an organism-derived substance, or may be a chemically synthesized synthetic substance. Specific examples of the test substance include nucleic acid molecules such as siRNA, shRNA, dsRNA, miRNA, antisense DNA, antisense RNA and aptamers, and the like.

### 4-2. Step (A)

In the screening method of the present invention, first, in the step (A), a test substance is brought into contact with a cell expressing RBM7.

The cell expressing RBM7 is not particularly limited as long as it expresses RBM7, but for example, a cell that endogenously expresses RBM7 such as a fibrotic cell, a cell that have acquired RBM7 expression ability by transfecting a RBM7 gene into a normal cell or the like can be used. The transfection of RBM7 gene into a normal cell can be performed using a known genetic engineering technique.

In order to bring a test substance into contact with a cell expressing RBM7, the cell and the test substance may be added to a medium in which the cell can grow, and the cell may be cultured at a temperature at which the cell can grow. Further, when using a nucleic acid molecule such as siRNA, shRNA, dsRNA, miRNA, antisense DNA or antisense RNA as a test substance, a nucleic acid introduction auxiliary agent also may coexist so that these nucleic acid molecules are easily introduced into the cell. Examples of the nucleic acid introduction auxiliary agent examples of nucleic acid-introduction agent include lipofectamine, oligofectamine, RNAifect, liposome, polyamine, DEAE dextran, calcium phosphate, dendrimer, and the like.

### 4-3. Step (B)

Next, in the step (B), the expression level of RBM7 in the cell is measured. The RBM7 expression level in the cell can be measured by measuring mRNA level of intracellular RBM7 and/or RBM7 level (protein level) according to a known method.

### 4-4. Step (C)

Furthermore, in the step (C), a test substance is selected as a candidate for an active ingredient of an antifibrotic agent when ability to suppress expression of RBM7 is recognized.

Specifically, it is determined that a test substance whose expression level of RBM7 is low, as compared to the expression level of RBM7 in the case of performing the step (A) and the step (B) without adding the test substance, has an ability to suppress expression of RBM7.

A test substance in which an ability to suppress expression of RBM7 has been recognized has an action of inhibiting fibrosis and is selected as a candidate for the active ingredient of the antifibrotic agent. The test substance thus selected can actually be tested for its fibrosis-inhibiting effect and evaluated for its clinical application as an antifibrotic agent.

### 5. Antifibrotic agent

The antifibrotic agent of the present invention is characterized by using a substance that suppresses expression of RBM7 as an active ingredient.

### 5-1. Active ingredient

RBM7 is involved in excessive cell death in the fibrotic phase and has an action of promoting fibrosis. By suppressing the expression of RBM7, the antifibrotic agent of the present invention can suppress cell death in the fibrotic phase and effectively inhibit fibrosis. The amino acid sequence of RBM7 and the base sequence of mRNA encoding the same are as described in "1. Biomarker".

In the present invention, the "substance that suppresses expression of RBM7" is not particularly limited as long as it is pharmaceutically acceptable and can suppress the expression of RBM7 from DNA encoding RBM7 (RBM7 gene). The substance that suppresses expression of RBM7 may be one that exhibits a suppressive effect on the expression of RBM7 at any stage such as RBM7 gene transcription, post-transcriptional regulation, translation, post-translational modification, or the like. Specific examples of the substance that suppresses expression of RBM7 include nucleic acid medicines such as nucleic acid molecules that suppress transcription of RBM7 gene such as decoy nucleic acid; RNA molecules having an RNA interference action on the mRNA of RBM7 such as siRNA, shRNA or dsRNA or precursors thereof; nucleic acid molecules that suppress translation of mRNA of RBM7 such as miRNA, antisense nucleic acids (antisense DNA, antisense RNA) and ribozymes, and the like. These nucleic acid medicines may be used alone or in combination of two or more kinds. In addition, the base sequence of these nucleic acid medicines can be appropriately designed by a method known to those skilled in the art based on information on the base sequence of the RBM7 gene. Among these nucleic acid medicines, from the viewpoint of easiness for clinical application and the like, examples thereof include preferably siRNA, shRNA, dsRNA, miRNA and antisense nucleic acid, further preferably siRNA and shRNA, and particularly preferably siRNA.

Moreover, when the nucleic acid molecule is an RNA molecule, it may be designed so as to be produced in vivo. Specifically, a DNA encoding the RNA molecule may be inserted into an expression vector for mammalian cells. Examples of such expression vector include viral vectors such as retrovirus, lentivirus, adenovirus, adeno-associated virus, herpes virus and Sendai virus, animal cell expression plasmids, and the like.

### 5-2. Target disease

Since RBM7 promotes fibrosis due to excessive cell death in the fibrotic phase, the antifibrotic agent of the present invention can inhibit fibrosis by suppressing the expression of RBM7. Therefore, the antifibrotic agent of the present invention can be used for treatment, prevention or progression prevention of fibrosis. RBM7-promoted cell death commonly functions in any organ. Therefore, fibrosis to which the antifibrotic agent of the present invention is applied may be fibrosis in any organ. Specific examples include pulmonary fibrosis, hepatic fibrosis, renal fibrosis, scleroderma, cardiac fibrosis, myelofibrosis, and the like.

Further, the antifibrotic agent of the present invention can be used not only for humans but also for mammals such as cows, pigs, dogs, cats, goats, rats, mice and rabbits, and is preferably used as a pharmaceutical for humans.

### 5-3. Mode of administration

An administration form of the antifibrotic agent of the present invention may be either oral administration or parenteral administration as long as an anti-fibrotic effect can be obtained, and may be appropriately set according to a type of active ingredient used. Specific examples of the administration form of the antifibrotic agent of the present invention include oral administration; and parenteral administration such as injection administration (intravenous injection, subcutaneous injection, intramuscular injection, intraperitoneal injection, local injection to an affected area, etc.), and suppository administration.

A dose of the antifibrotic agent of the present invention may be appropriately set according to the type of active ingredient used, administration form, organ to be applied, degree of symptom of patient, and the like. For example, when a nucleic acid molecule is used as the active ingredient, as a single dose of the nucleic acid molecule, for example, about 0.01 µg to 3000 mg/kg body weight may be administered once every 1 to 3 days, and it can be appropriately adjusted according to a pharmacological action, blood concentration, side effects and the like, for each antifibrotic agent and administration subject.

### 5-4. Preparation form

The antifibrotic agent of the present invention is prepared in a preparation form according to the type of active ingredient and administration form. Examples of the preparation form of the antifibrotic agent of the present invention include solid preparations such as tablets, capsules, pills, powders, granules and suppositories; liquid preparations such as solutions, suspensions, emulsions and injections; and the like.

Moreover, the antifibrotic agent of the present invention is formulated by adding a pharmaceutically acceptable carrier or additive according to the preparation form. For example, in the case of a solid preparation, it can be formulated using an excipient, a binder, a disintegrating agent, a lubricant, or the like. Further, in the case of a liquid preparation, it can be formulated using physiological saline, a buffer solution, or the like.

Furthermore, in the case of using a nucleic acid molecule as an active ingredient in the antifibrotic agent of the present invention, it is desirable that the nucleic acid molecule is formulated together with a nucleic acid introduction auxiliary agent so that the nucleic acid molecule is easily transferred into a cell. The nucleic acid introduction auxiliary agent is as described in "4. Method for screening active ingredient of antifibrotic agent" above.

### 6. Method for diagnosing fibrosis

The present invention provides a method for diagnosing fibrosis. The diagnostic method is characterized by including a step of detecting RBM7 in a biological sample collected from a test subject, and a step of diagnosing fibrosis based on a result obtained in the above step.

In the diagnostic method of the present invention, the antibody detection method, criteria for determining that the test subject has fibrosis and the like are as described in "2. Method for detecting fibrosis" above.

### 7. Method for treating, preventing or preventing progression of fibrosis

The present invention provides a method for treating, preventing or preventing progression of fibrosis (hereinafter referred to as a treatment method and the like). The treatment method and the like are characterized by including a step of administering to a fibrosis patient a therapeutically effective amount of a substance that suppresses expression of RBM7.

In the therapeutic method and the like of the present invention, the substance that suppresses expression of RBM7 to be administered, therapeutically effective amount, administration method and the like are as described in "5. Antifibrotic agent" above.

### EXAMPLES

Hereinafter, the present invention will be described in more detail with reference to examples, but the present invention is not limited to these examples.

### [Animal]

Mice (males younger than 6 weeks of age) were individually housed in a ventilated cage in a temperature-controlled room in a facility free of specific pathogens and provided free access to food and water. All experiments were approved by a local ethics committee and carried out in accordance with the guidelines of the Animal Care and Use Committee of the Research Institute for Microbial Diseases, Osaka University.

### [Reagent]

As antibodies for flow cytometry, following commercial products were purchased and used: anti-Ly6C (AL-21; BioLegend), anti-F4/80 (BM8; BioLegend, San Diego, CA, USA), anti-CD45 (30-F11; BioLegend), anti-CD66a (Ceacam1) (Mab-CC1; BioLegend), and anti-Msrl (LS-c43466; LSBio).

As antibodies for Western blotting, immunoprecipitation and immunohistochemical staining, following were used: anti-BCLXL (No. 2762; Cell Signaling Technology, Danvers, MA, USA), anti-BCL2 (No. 2876; Cell Signaling Technology), anti-PARP (no. 9532; Cell Signaling Technology), anti-human cleaved PARP, anti-mouse cleaved PARP (No. 9544; Cell Signaling Technology), anti-yH2AX (No. 9718; Cell Signaling Technology), anti-ATM (Nos. 2618 and 32420; Abcam, anti-BRCA1 (16780 and 16781; Abcam), anti-BRCA2 (sc-8326; Santa Cruz Biotechnology, Dallas, TX, USA), anti-ATRA (No. 2905; Abcam) anti-mouse RBM7 (preparation), anti-histone H3 (No. 9715; Cell Signaling Technology), anti-human RBM7 (No HPA013993; Atlas Antibodies, Bromma, Sweden)), anti-Oasla (sc-365357, Santa Cruz Biotechnology), anti-Oasla (Santa Cruz Biotechnology), anti-Oasla anti-PPO1R14B (No. 18476-1-AP; Proteintech, Rosemont, IL, USA), anti-PCOLCE (no. ab39204; Abcam), anti-PCOLCE-β-actin (no. sc-1615; Santa Cruz Biotechnology), mouse monoclonal anti-a smooth muscle actin (no. A254 7; Sigma, St. Louis, MO, USA), horseradish peroxidase (HRP)-conjugated anti-mouse IgG (no. NA931V; GE Healthcare), HRP-conjugated anti-rabbit IgG (NA934V number; GE Healthcare, Little Chalfont, UK), Alexa Fluor 488-conjugated goat anti-rabbit IgG (A21202, Life Technologies, Carlsbad, CA, USA) Alexa Fluor 568-conjugated goat anti-mouse IgG (No 11031; Life Technologies) and normal mouse IgG (no. sc-2025; Santa Cruz).

### [Animal]

Rbm7-/- mice were generated using gene targeting technology and genotyped by PCR as described in Non-Patent Document 7. After backcrossing to C57BL/6J mice 10 times or more, Rbm7+/+ (WT) and Rbm7-/- litters were obtained by crossing the Rbm7+/- mice. Cebpb -/- chimeric mice were prepared based on the description in Non-Patent Document 11.

### [Human clinical sample]

Lung tissue was collected from 9 cases of chronic fibrotic idiopathic interstitial pneumonia (6 cases of idiopathic pulmonary fibrosis and 3 cases of fibrotic non-specific interstitial pneumonia) and 9 cases of non-fibrotic normal lung area affected by other pathological conditions such as cancer. Experienced pathologists confirmed that all samples analyzed in this study were free of carcinoma. All patients and control subjects signed a consent form approved by the Ethics Committee of Osaka University. Human study was approved by the Ethics Committee of Osaka University.

### [Fibrosis model]

For experiments of pulmonary fibrosis, a group of 8 to 10-week-old mice of the same sex was anesthetized, and PBS or bleomycin (BLM, 3 µg/g body weight; Nippon Kayaku, Tokyo, Japan) was intratracheally injected, lungs were collected at predetermined time points. For experiments of hepatic fibrosis, a group of sex-matched 8-12-week-old mice was fed a normal diet or choline-deficient L-amino acid high-fat diet (CDA-HFD; Research Diets, New Brunswick, NJ, USA), livers were collected after 12 weeks. For experiments of renal fibrosis, unilateral ureteral obstruction (UUO) was induced by left ureteral ligation in 6-8-week-old male mice. A contralateral kidney served as a control. Kidneys were collected on day 7 after surgery. In addition, pan-caspase inhibitor Q-VD-OPh (No. 1135695-98-5, Santa Cruz Biotechnology) was injected intraperitoneally at a concentration of 20 mg per kg body weight on day 5, and injected intraperitoneally at a concentration of 10 mg per kg body weight on day 6 to 8 after the bleomycin inoculation.

### [In vivo lung-specific siRNA]

Lung-specific siRNA-mediated targeted RNA knockdown was obtained by injection of Invivofectamine (registered trademark) Lung-siRNA complex via a dorsal vein. The injection was performed at a concentration of siRNA of 0.5 mg/kg body weight. Invivofectamine (registered trademark) Lung was provided by Thermo Fisher Scientific (Waltham, MA, USA). Ambion (registered trademark) In Vivo Predesigned siRNA was purchased from Thermo Fisher Scientific (Rbm7; S84280, NEAT1; n256283). Invivofectamine (registered trademark) Lung-siRNA complex was prepared according to a protocol. Specifically, a siRNA solution was mixed with a complex formation buffer, and Invivofectamine (registered trademark) Lung Reagent was added thereto. The Invivofectamine (registered trademark) Lung-siRNA mixture was dialyzed on a Float-A-Lyzer (registered trademark) dialyzer (8-10 kDa) according to a protocol. After dialysis, a final volume was adjusted and diluted with PBS to a predetermined concentration.

### [Primary cell isolation and fluorescence-activated cell sorting]

To isolate primary cells, lung was excised from mice and cut into approximately 1 mm³ fragments. Lung primary cells were incubated at 37°C for 1 hour, in the presence of penicillin (100 U/ml)-streptomycin (100 µg/ml) (no. 09367-34; Nacalai Tesque) in F12K medium (no. 21127022; Thermo Fisher Scientific), to be isolated from lung tissue by collagenase I (1 mg/ml; Sigma) Kyoto, Japan). The cells were subsequently centrifuged at 1100 rpm for 10 minutes and treated with F12K containing 0.2 mg/ml deoxyribonuclease I (no. 11284932001; Roche) at 25°C for 5 minutes. The single cell suspension was then centrifuged at 1100 rpm for 10 minutes and treated with erythrocyte lysis buffer (Sigma-Aldrich) for 3 minutes. The cells were stained with anti-mouse CD45-APC (BioLegend), and then CD45 and CD45-cells were sorted using FACS Aria (BD Biosciences, San Jose, CA, USA). For isolation of primary lung epithelial cells, the cells were resuspended in F-12K medium containing 10% FBS and placed in a 10 cm culture dish at 37°C for 2 hours. After incubation, non-adherent cells were collected.

### [Hydroxyproline assay]

A tissue sample was subjected to base hydrolysis. Hydroxyproline was measured using a hydroxyproline colorimetric assay kit (BioVision, San Francisco, CA, USA) according to a protocol, and levels were expressed as µg/g tissue.

### [SatM Migration assay]

SatM was harvested from bone marrow according to the method described in Non-Patent Document 11. Migration ability of cells was evaluated using a migration chamber (No. 3421; Corning, NY, USA) having an insert of 5 µm pore size according to a protocol. Specifically, inserts were placed in 24-well plates containing RPMI 1640 containing 5% (v/v) fetal bovine serum (No. 10270-106; Thermo Fisher Scientific) with homogenized lung or cell extracts. A PBS or BLM inoculated mouse lung was homogenized with ULTRA-TURRAX (registered trademark) T25 (IKA, Werke Staufen, Germany), and an insoluble fraction was removed by centrifugation. The same number of cells were homogenized with BioMasher III (Nippi, Tokyo, Japan), and an insoluble fraction was removed by centrifugation. A supernatant of the homogenized sample was collected and concentrated by ultrafiltration with Amicon (registered trademark) Ultra-15 10K (no. UFC901024; Millipore) according to a protocol. Serum starved cells (n = 1 × 10⁵) were then placed on the inserts and incubated at 37°C for 16 hours. Migrated cells were stained with Diff Quick (No. 16920, Sysmex, Hyogo, Japan) and quantified by CellTiter-Glo (registered trademark) Luminescent Cell Viability Assay (No. G7570, Promega, Madison, USA) according to a protocol.

### [Western blotting]

Proteins were separated by SDS-PAGE and transferred to a polyvinylidene difluoride (PVDF) membrane. The membrane was incubated with an appropriate primary antibody and a HRP-conjugated secondary antibody, and blots were visualized using enhanced chemiluminescence (ECL). β-actin was probed as a loading control. The following primary antibodies were used: rabbit polyclonal anti-yH2AX (1 : 1000; Cell Signaling Technology), rabbit polyclonal anti-PARP (1 : 1000; Cell Signaling Technology), rabbit polyclonal anti-cleavage PARP (1 : 1000; Cell Signaling Technology), rabbit polyclonal anti-BCL2 (1 : 1000; Cell Signaling Technology), rabbit polyclonal anti-BCLXL (1 : 1000; Cell Signaling Technology), rabbit polyclonal anti-cleavage caspase 3 (1 : 1000; Cell Signaling Technology) rabbit anti-mouse RBM7 (1 : 1000; prepared independently) and rabbit polyclonal anti-human RBM7 (1 : 200; Atlas antibody) were used.

### [RT-qPCR]

Total RNA was isolated using a High Pure RNA isolation kit (no. 1828665; Roche) or TRIzol (no. 5596018; Thermo Fisher Scientific) according to a protocol. RNA was quantified with NanoDrop 1000 and reverse transcribed using ReverTra Ace qPCR RT Master Mix (Toyobo, Osaka, Japan). Quantitative reverse transcription PCR (RT-qPCR) was performed using SYBR Green 2x Master Mix according to a protocol of ReverTra Ace qPCR RT Master Mix, or performed using TaqMan Gene Expression Assay (Thermo Fisher Scientific) for tnfa, il6, il1b, cxcl1, tgfb, collal, and 18S. Relative mRNA expression values were calculated using a standard curve method with 18S as a target gene.

Primer sequences in qRT-PCR are as follows.
human NEAT1(fw: atgccacaacgcagattgat, rv: cgagaaacgcacaagaagg)
human NEAT1.2 (fw: ctagaggctcgcattgtgtg, rv: gcccacacgaaaccttacat)
human MALAT1 (fw: cttccctaggggatttcagg, rv: gcccacaggaacaagtccta)
mouse NEAT1 (fw: ttgggacagtggacgtgtgg, rv: tcaagtgccagcagacagca)
mouse MALAT1 (fw: tcggccttgtagattaaaacgaa, rv: aacggccgtcaacttaacct)
18S (fw: cgaacgtctgccctatcaactt, rv: acccgtggtcaccatggta),
NIFK-AS1 (fw: cttcccaggctcagagatgg, rv: cctgatgcggcaaatctgtgg)
HEXA-AS1 (fw: ggcttgagaagtgatcaagg, rv: tatcatgcccagtgaaagcc)
ZNF197-AS1 (fw: gatttgcatttcaattgagg, rv: attctgttctcatctgcagg)
ENO1-AS1 (fw: acctacctggattcttgtgaaccattc, rv: cttgaactttgagaaattcc)
FAM41C (fw: gaacattgctgtaaactgctctgaga, rv: caaggtcctcttgctgttcc)
HCG25 (fw: gagcctaagttcaaacgagg, rv: ttcccaagaccacttcccat)
TMEM9B-AS1 (fw: ggtcttctactggacaaagg, rv: tattccagaacagcacttcc)
proSTK11IP (fw: gggagtctaaggaaaaggag, rv: cagtgaaaggagagcgtatc)
proPOGZ (fw: agttccaagaaaccacacac, rv: ggtcgtttgagtggactaac)
STK11IP (fw: ggtcagacacaaccacaagg, rv: cgtgaccatggtagttggag)
POGZ (fw: aagcctggagagatgcctta, rv: aatgcattgccatttttgaa)

### [TUNEL Assay]

TUNEL Staining of paraffin-embedded tissue sections was performed using ApopTag (registered trademark) Peroxidase In Situ Apoptosis Detection Kit according to a protocol. Sections were stained with diaminobenzidine and counterstained with hematoxylin. TUNEL-Positive apoptotic cells showed brown nuclei. Five views were randomly selected in each section (×20 magnification), and the number of apoptotic cells was calculated.

### [Cell death assay]

Cells were cultured on glass chamber slides with or without 50 µM etoposide (no. E0675; Tokyokasei, Tokyo, Japan) for 24 hours, and live/death detection kit (PK-CA707-30002; PromoKine, Heidelberg, Germany) was used to detect cell death according to a protocol.

### [Separation and analysis of bronchoalveolar fluid (BALF) by flow cytometry and ELISA]

Mice were euthanized with CO₂ anesthesia, a trachea was cannulated, and lungs were washed 3 times with 1 ml of ice-cold PBS (3 ml total). After centrifugation, cells were treated with erythrocyte lysis buffer (Sigma-Aldrich) for 3 minutes. After washing with ice-cold FACS buffer [0.5% bovine serum albumin (BSA) in PBS and 2 mM EDTA, pH 7.2], cells were incubated with antibody for 30 minutes and washed twice with FACS buffer. Data were acquired using a flow cytometer (FACSCalibur, BD Biosciences) and analyzed by FlowJo software (Tree Star). A commercial ELISA (R&D Biosystems, Minneapolis, MN, USA) was used to measure total TNF-α and IL-6 in samples detected according to a protocol.

### [Histological analysis]

Lung, liver and kidney tissues were fixed in 4% paraformaldehyde (PFA) in PBS for 24 hours and embedded in paraffin. Sections (4 µm thick) were cut using a paraffin microtome with a stainless steel knife, mounted on glass slides, deparaffinized with xylene, stepwise dehydrated with ethanol, and stained with hematoxylin and eosin. Sections were stained with Azan to assess collagen deposition. To assess collagen fibril formation in the kidney, sections were stained using 0.2% phosphomolybdic acid hydrate, 0.4% Direct Red 80 and 1.3% 2,4,6-trinitrophenol (Polysciences, Inc., Warrington, PA, USA) by Picrosirius Red method. For immunofluorescence studies, lung tissue was fixed on ice in 4% PFA for 1 hour and then incubated overnight at 4°C in PBS containing 30% sucrose. The tissue was embedded in OCT compound (Sakura Finetek, Torrance, CA, USA), cryosectioned into 10 µm sections and mounted on Superfrost slides (Thermo Fisher Scientific). The sections were washed with PBS, blocked with 3% BSA, and incubated with mouse monoclonal α smooth muscle actin (1 : 200). A secondary antibody used was FITC-conjugated (1 : 1000; Life Technologies). For Oil Red O staining, liver tissue was fixed with 4% paraformaldehyde, frozen in OCT, and sectioned. After 15 minutes of air drying, sections were stained with Oil Red O (no. 01391; Sigma) and counterstained with hematoxylin.

### [Immunohistochemistry of tissue section]

Formalin-fixed paraffin-embedded 4 µm thick (lung and liver) and 3 µm thick (kidney) sections were stained using standard procedures. Specifically, the sections were deparaffinized with alcohol stepwise and washed with PBS. Heat activated antigen recovery was performed in a Pascal Pressurized Heating Chamber (Dako, Glostrup, Denmark) using citrate buffer (no. S170084-2; DAKO) (lung and liver) and autoclave (kidney). Endogenous peroxidase was blocked with a peroxidase blocker (DAKO) (lung and liver) and H₂O₂ (kidney). Slides were blocked for non-specific protein binding by incubation in 3% BSA (lung and liver) and 5% normal goat serum (kidney). Tissues were immunostained with rabbit anti-human RBM7 or rabbit anti-mouse RBM7 at both a 1 : 300 dilution (lung and liver) and a 1 : 1000 dilution (kidney).

### [Immunocytochemistry and in situ hybridization of cultured cells]

Cells were cultured on coverslips, fixed with 4% PFA, clarified with 0.5% Triton X-100 in PBS and blocked with 1% BSA. The cells were then incubated with primary and secondary antibodies for 1 hour and 30 minutes, respectively. The antibodies were used at following dilutions: rabbit polyclonal anti-γ-H2AX, 1 : 500; rabbit polyclonal anti-human RBM7, 1 : 200; Alexa Fluor 488-conjugated goat anti-rabbit IgG, 1 : 1000; and Alexa Fluor 568-conjugated goat anti-mouse IgG (1 : 1000). QuantiGene ViewRNA ISH cell assay (Thermo Fisher Scientific) using a QG ViewRNA TYPE 1 probe set designed to target human NEAT1 (VA1-12621-01; Thermo Fisher Scientific) or mouse NEAT1 (VB1-12456; Thermo Fisher Scientific) was used for in situ hybridization (ISH) according to a protocol. For co-immunofluorescence, following primary antibodies and dilutions were used after FIH experiment: rabbit polyclonal anti-human RBM7 (1 : 200; Atlas Antibodies), mouse monoclonal anti-BRCA1 (ab16781, 1 : 200; Abcam), rabbit polyclonal anti-BRCA2 (sc-8326, 1 : 50; Santa Cruz), mouse polyclonal anti-ATM (1 : 250; Abcam), rabbit monoclonal anti-ATR (1 : 100; Abcam) or rabbit polyclonal anti-p53 (1 : 250; Abcam) 2000; Cell Signaling Technology). Also, Alexa 488-conjugated secondary antibody (1 : 1000; Life Technologies) was used. Genomic DNA was stained with Hoechst 33342 dye. Samples were examined using a LSM780 confocal laser scanning microscope (Carl Zeiss, Oberkochen, Germany). NEAT1-containing paraspeckle was identified by NEAT1-containing nuclear aggregates. Quantification of NEAT1-containing paraspeckle was performed by acquiring 5 images at ×63 magnification and counting the number of nuclei with NEAT1-containing paraspeckle relative to the total number of nuclei.

### [Creation of bone marrow chimeric mouse]

Bone marrow (BM) cells were prepared from WT and Rbm7-/- mice and intravenously injected into lethally γ-irradiated CD45.1+ donor mice. Chimeric mice were administered neomycin and polymyxin B (Sigma) in drinking water for 4 weeks. The mice were reconstituted for at least 8 weeks before analysis. 90% or more of splenocytes derived from the chimeric mice were CD45.2+.

### [Targeting deletion of Rbm7 mediated by CRISPR/Cas9]

A target sequence was cloned into plasmid pX330 for genome editing using CRISPR/Cas9. Purified plasmids (Rbm7 and empty vector control) were transfected into HEK293 cells using Lipofectamine 2000. A single clone was selected and sequenced. Disappearance of RBM7 protein was confirmed by Western blotting.

### [HEK293 and HEK293 T cell culture]

Cells were cultured at 37°C in a DMEM (no. 08459-64; Nacalai Tesque) medium containing 50 µM 2-mercaptoethanol (no. 21417-52; Nacalai Tesque), 10% (v/v) fetal bovine serum (no. 10270-106; Gibco), 100 U/ml penicillin G and 100 µg/ml streptomycin (both manufactured by Nacalai Tesque) in a humidified 5% CO₂ atmosphere.

### [Plasmids and primers]

Full length human Rbm7 lacking RNA-binding domains (1-267 and 92-267) and cDNAs encoding Rbm7 were inserted into a pFlag-CMV2 (Sigma) or pcDNA 3.1(+) (Invitrogen) vector. Human full length BRCA1 was purchased from addgene (No. 71117).

### [Transfection of HEK293 and HEK293T cells]

Cells were seeded a day before transfection. A next day, when the cells were 70-80% confluent, a medium was replaced with a medium without penicillin and streptomycin. DNA in Opti-MEM (Life Technologies) was mixed with Lipofectamine 2000 (Invitrogen) and incubated at room temperature for 20 minutes or more. The mixture was then added dropwise to the cells. After 6 hours, the medium was replaced with complete medium, and the cells were used for experiments 24 hours after transfection.

### [RNA immunoprecipitation (RIP) of Flag-tagged RBM7-expressing HEK293 cells]

FLAG-labeled RBM7-expressing HEK293 cells were lysed in cell lysis buffer [25 mM Tris (pH 7.6), 150 mM NaCl, 1 mM EDTA, 0.5% NP-40, 1 mM DTT, 150 U/ml RNaseOUT RNase inhibitor (Thermo Fisher Scientific) and Protease Inhibitor Cocktail (Roche) without EDTA]. The lysate was clarified by centrifugation. The lysate was incubated on anti-FLAG M2 agarose affinity gel (No. A2220; Sigma) for 2 hours. An immunoprecipitate was finally washed 3 times with cell lysis buffer and eluted with Trizol (No. 15596018; Invitrogen). Isolated RNA was treated with DNasel (No. 79254; QIAGEN).

### [RNA Sequencing]

RNA eluted from the RIP sample (n = 2 biological replicates) and total RNA from WT and Rbm7-/- HEK293 cells treated with or without 50 µM etoposide (Tokyokasei) (4 µg/sample) Ribo-Zero Gold rRNA removal kit (Illumina, San Diego, CA, USA) was used to deplete ribosomal RNA. A library was prepared with TruSeq Stranded mRNA LT Sample Prep Kit (Illumina) and sequenced on Illumina HiSeq 2500 platform with 100-bp paired raw passing filter reads and 500,000-600,000 reads.

### [Magnetic resonance image]

Images from MRI were acquired and processed using Avance III BioSpec 117/11 system with 1H QD (Bruker, Ettlingen, Germany) as described in Non-Patent Document 11. Mice were anesthetized with sevoflurane during MRI. A fast spin echo protocol (RARE) was used for T1-weighted and T2-weighted imaging and water suppression imaging. Acquired images were converted to DICOM format and processed for 3D reconstruction using ImageJ software (National Institutes of Health, Bethesda, MD, USA). Final images were processed using Imaris software (Bitplane AG, Zurich, Switzerland), and a fibrotic area was surrounded in gray for indication. Parameters for imaging are as follows: T1 weighted: TR/TE = 800/12 ms, 10 times average; T2 weighted: TR/TE = 3500/32 ms, 8 times average; and water suppression: suppression pulse offset from water signal -same as T1 weighted by 300 Hz. The image acquisition matrix was 256 × 128, a field of view was 4 × 3 cm, and 18 sheets each with a thickness of 1.2 mm were obtained. Fat suppression was applied to all imaging methods.

### [Mapping and counting of sequence reads]

Sequence reads were filtered with cutadapt and were aligned on human genome hg38 (ftp://ussd-ftp.illumina.com/Homo#sapiens/UCSC/hg38/Homo# sapiens#UCSC#hg38.tar.gz) using Bowtie2 v2.2.9 as a part of TopHat v2.1.1. FPKM data was obtained using Cufflinks with GENCODE GTF annotation file.

### [FPKM Data plot and pathway analysis]

The FPKM data was imported into Excel and processed to plot a heat map. The Excel file was also used for standard pathway analysis by IPA (Qiagen). FPKM data of apoptosis-related genes selected based on a pathway map (Cell Signaling Technology) were plotted on a scatter plot.

### [Preparation of shRNA against various types of long non-coding RNA and BRCA1]

Two or more effective shRNA clones for each target coding sequence were prepared. The target sequences are as follows:
Human NEAT1 coding sequences (5'-gaggaagttctccaatgtt-3', 5'-gtcttagaaaagccttgta-3')
Human MALAT1 coding sequences (5'-ggggattcttctctaatct-3', 5'-agaaagtcaggggtctata-3')
Human BRCA1 coding sequences (5'-aagtctattaaagaaagaaaa-3', 5'-aatgatgaatattactaatag-3 ')

The shRNA sequence was cloned into lentivirus expression plasmid pFU6pGKpuro and transfected into HEK293T cells to produce recombinant lentivirus. WT or Rbm7-/- HEK293 cells were transduced with lentiviral supernatant in the presence of polybrene (Millipore) and selected with 1 µM puromycin (InvivoGen, San Diego, CA, USA). RT-qPCR and Western blot analysis were performed to confirm significant depletion of each target sequence.

### [Formaldehyde-crosslinked RIP]

HEK293 cells were fixed with 0.1% formaldehyde in PBS to crosslink a protein-RNA complex and glycine was added at a concentration of 0.25 M to quench formaldehyde. The cells were then washed with PBS, and incubated on ice with 1 mL of RIPA buffer [50 mM Tris?Cl, pH 7.5, 1% Nonidet P- 40 (NP-40), 0.5% sodium deoxycholate, 1 mM EDTA, 150 mM NaCl], 1 mM DTT, a protease inhibitor and RNaseOUT for 15 minutes. The lysate was then sonicated using Bioruptor (Diagenode). Insoluble components were removed by microcentrifuging at 10,000 rpm at 4°C for 10 minutes. Dynabeads coated with specific antibody [BRCA1 (2 µg; Abcam), BRCA2 (2 µg; Santa Cruz), ATM (2 µg; Abcam), ATR (2 µg; Abcam), p53 (5 µl; Cell Signaling Technology), PARP (5 µl; Cell Signaling Technology) or IgG (2 µg; Santa Cruz)] were added to the lysate and incubated at 4°C for 3 hours. The beads were washed 4 times with RIPA buffer. Finally, decrosslinking was performed, and the sample was eluted with Trizol (no. 15596018; Invitrogen). Isolated RNA was treated with DNase1 (QIAGEN), and cDNA synthesis was performed using ReverTra Ace qPCR RT master mix (FSQ-201; TOYOBO).

### [UV-Crosslinked RNA immunoprecipitation (CLIP)-qPCR]

For UV crosslinking, FLAG-labeled RBM7- and BRCA1-expressing HEK293 cells were washed twice with PBS and irradiated at 2000 J/m² to crosslink the protein-RNA complex. The cells were then washed with PBS, and incubated on ice with 500 µl of RIPA buffer [50 mM Tris-HCl, pH 7.5, 1% Nonidet P-40 (NP-40), 0.5% sodium deoxycholate, 1 mM EDTA, 150 mM NaCl], 1 mM DTT, a protease inhibitor and RNaseOUT for 15 minutes. The lysate was then sonicated using Bioruptor (Diagenode). Insoluble components were removed by microcentrifuging at 10,000 rpm at 4°C for 10 minutes. The lysate was incubated with an anti-FLAG M2 agarose affinity gel (no. A2220; Sigma) at 4°C for 2 hours. Beads were washed with high salt buffer (20 mM Tris-HCl pH 7.6, 500 mM NaCl, 1% NP40, 1 mM EDTA) twice, and with low salt buffer (20 mM Tris-HCl pH 7.6, 500 mM NaCl, 1% NP40, 1 mM EDTA). The beads were suspended in 200 µl of TE containing 0.5% SDS and 2 µl of proteinase K (NEB) and incubated at 55°C for 1 hour. RNA was extracted with Trizol (Invitrogen).

### [RNA Antisense purification (RAP)]

Probe design and generation: In order to make a probe used to capture target RNA, a 90-mer DNA oligonucleotide (Fasmac, Kanagawa, Japan) spanning an entire length of the target RNA was designed and synthesized. A sequence of each DNA oligonucleotide probe was antisense to a complementary strand of the target RNA sequence. Each DNA oligonucleotide probe was also modified with 5' biotin to allow capture of DNA/RNA hybrids on streptavidin-coated magnetic beads. For UV crosslinking, 20 million HEK293 cells were washed twice with PBS and irradiated at 8000 J/m² to crosslink the protein-RNA complex. The cells were then washed with PBS and lysed in 866 µl of whole cell lysis buffer [10 mM Tris pH 7.5, 500 mM LiCl, 0.5% dodecyl maltoside (DDM; Sigma), 0.2% sodium dodecyl sulfate (SDS; Thermo Fisher Scientific) 0.1% sodium deoxycholate (Sigma) protease inhibitor], 920 U of Murine RNase Inhibitor (NEB) was added, and the sample was incubated on ice for 10 minutes to allow lysis to proceed. During this incubation, the cell sample was passed 3 to 5 times with a 26 gauge needle attached to a 1 ml syringe to break a pellet and shear genomic DNA. Each sample was then sonicated using Bioruptor. A sample was cooled during sonication to prevent overheating of the lysate.

The sample was then treated with 2.5 mM MgCl₂, 0.5 mM CaCl₂ and 20 U TURBO DNase (Thermo Fisher Scientific) at 37°C for 10 minutes to digest the DNA. The sample was returned to ice and the reaction was immediately stopped by addition of 10 mM EDTA and 5 mM EGTA. A disulfide bond was reduced by addition of 2.5 mM tris-2-carboxyethyl)phosphine (TCEP), and then the sample was mixed with twice the lysate volume of LiCl/urea buffer (10 mM Tris pH 7.5, 5 mM EDTA, 500 mM LiCl, 0.5% DDM, 0.2% SDS, 0.1% deoxycholate, 4 M urea, 2.5 mM TCEP). The lysate was incubated on ice for 10 minutes and then clarified by centrifugation at 16,000 g for 10 minutes. The lysate was then used for RNA antisense purification of the crosslinked complex. The lysate from 20 million cells was used for each capture. The lysate sample was preliminarily clarified by incubation with washed streptavidin C1 magnetic beads at 37°C for 30 minutes with intermittent shaking at 1100 rpm with Eppendorf Thermomixer C (30 s mixing, 30 s off). The streptavidin beads were then magnetically separated from the lysate sample using a Dynamag magnet (Life Technologies). The beads used to clarify the lysate were discarded, and the lysate sample was transferred twice to a new tube to remove any traces of magnetic beads.

A biotinylated 90-mer DNA oligonucleotide probe specific to the RNA target of interest (2 µg in water per sample) was heat denatured at 85°C for 3 minutes and then snap cooled on ice. The probe was mixed with the preliminarily clarified lysate and incubated at 40°C for 2 hours with intermittent shaking (30 s shaking, 30 s off) using an Eppendorf thermomixer, and the probe was hybridized to capture the target RNA. A complex of a biotinylated DNA probe and the target RNA was bound to the streptavidin beads by incubating each sample with 60 µl of washed streptavidin-coated magnetic beads at 40°C for 30 minutes with intermittent shaking with Eppendorf Thermomixer C as described above. The beads with the captured complex were washed 6 times with LiCl/urea hybridization buffer at 40°C for 5 minutes to remove non-specific related proteins. After resuspending the beads in Western blot sample buffer and boiling for 5 minutes, they were magnetically separated from the sample using a Dynamag magnet. The sample was then subjected to SDS-PAGE and Western blot analysis.

### [Microarray analysis]

Total RNA was isolated from mouse lung CD45-cells 10 days after PBS or BLM inoculation using an RNA isolation kit (Roche). 10 ng of total RNA was fragmented using GeneChip WT PLUS Reagent Kit (no. 902280; Thermo Fisher Scientific) according to a protocol to synthesize labeled ssDNA. After hybridizing the product on a mouse expression microarray chip (Mouse Transcriptome Array 1.0; Thermo Fisher Scientific), the microarray chip was stained, washed and scanned according to the protocol. Background-adjusted robust multichip average expression values were calculated using Expression Console Software (Thermo Fisher Scientific) and Transcriptome Analysis Software (Thermo Fisher Scientific).

### [Promoter assay]

Each fragment of a promoter region of NEAT 1 (including 2 kb upstream of a coding region) was amplified by PCR and cloned into pGL3-Basic vector (Promega). Using Lipofectamine 2000 reagent (Qiagen), HEK293 cells cultured in a 96-well plate (4 × 10⁴ cells per well) were transfected together with 46 ng of 20 ng of reporter plasmid containing DNA, 20 ng of pcDNA3.1 vector with or without RBM7 inserted, and 6 ng of reference plasmid (pRL-CMV) (Promega). After 48 hours of incubation with or without nutlin 3a (Sigma), luciferase activity was measured using Dual-Glo Luciferase assay system (Promega).

### [Statistical analysis]

All quantitative data are presented as mean ± standard deviation (SD) of at least 3 independent experiments. Differences between groups were analyzed using Student's t test, and survival tests were analyzed using log rank test. Values of P < 0.05 were considered statistically significant.

### [Results]

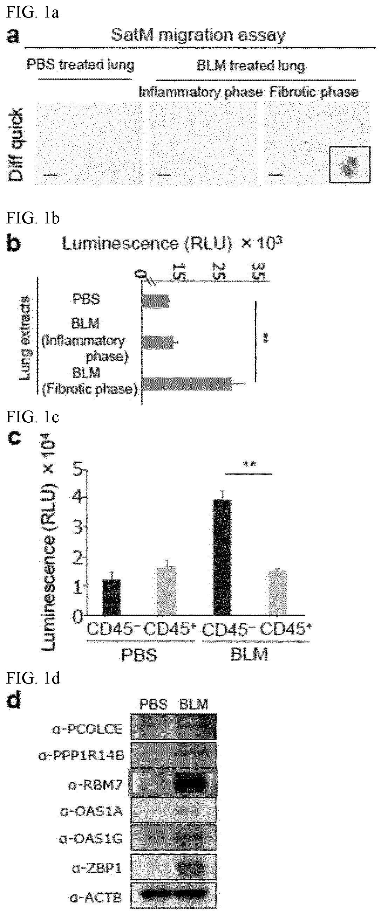

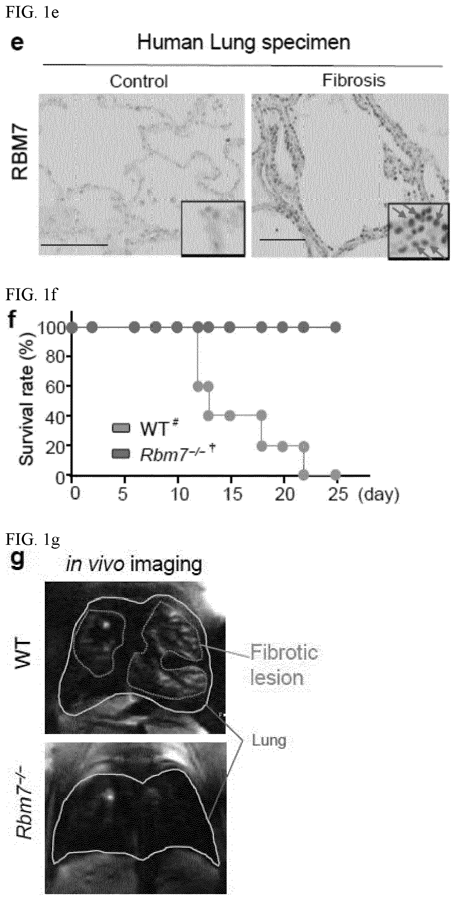

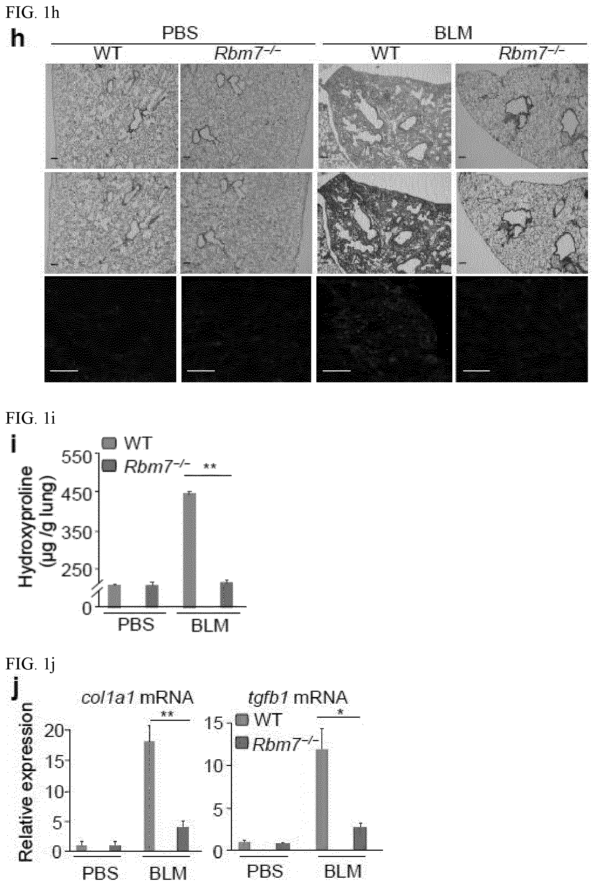

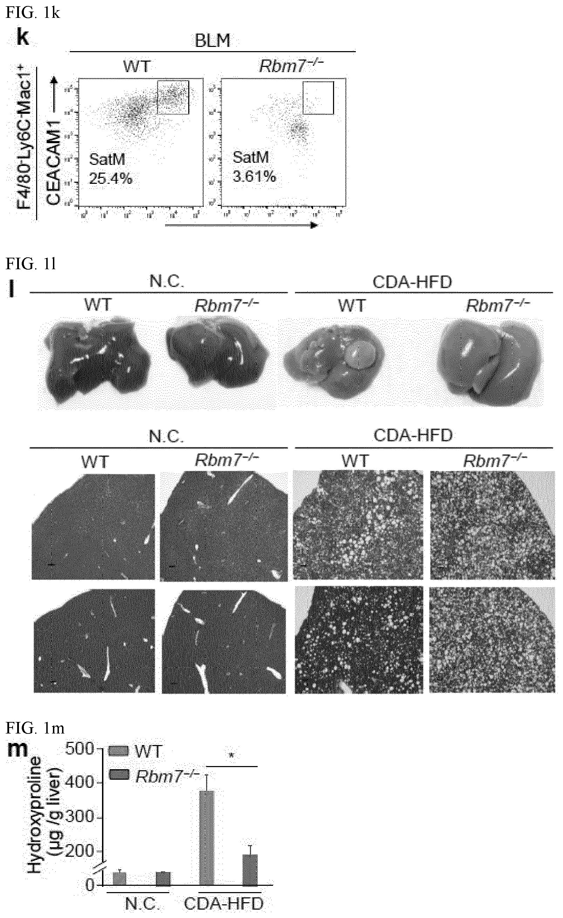

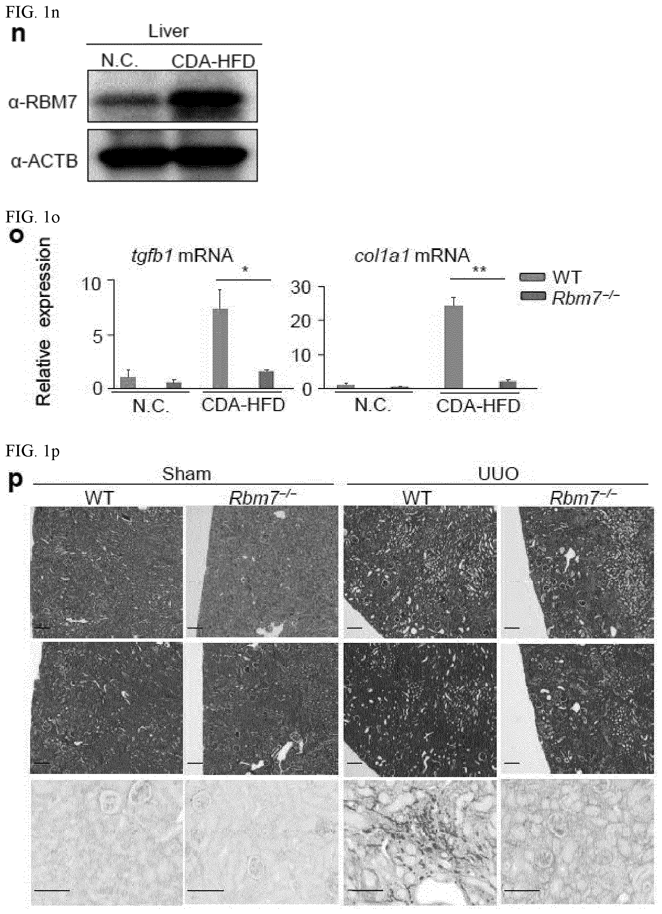

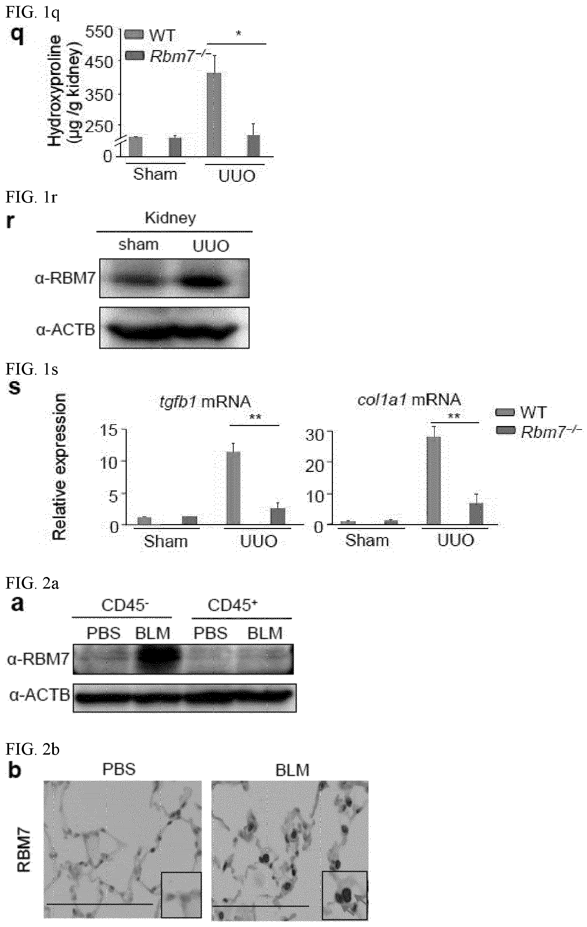

Figs. 1a to Is are results showing that Rbm7 deficiency confers resistance to fibrosis. * Indicates P < 0.05, and ** indicates P < 0.01 by Student's t test. Survival curves were analyzed by log rank test. All error bars represent SD. Similar results were also obtained in 3 (Fig. 1a to Fig. 1c), 5 (Fig. If, Fig. 1h to Fig. 1j) and 3 or more (Fig. Id, Fig. 1e, Fig. 1g, Fig. 1k to Fig. Is) experiments independently performed.

Fig. 1a to Fig. 1c are evaluation of migration ability of SatM in lungs treated with PBS or BLM (Fig. 1a, Fig. 1b), and evaluation of migration ability of hematopoietic cells (CD45+) and non-hematopoietic cells (CD45-) present in lung tissue (Fig. 1c). As shown in Fig. 1a to Fig. 1c, as a result of performing a cell migration assay using a lung extract in a fibrotic phase, SatM was significantly recruited with the extract in the fibrotic phase not in an inflammatory phase, from the lung of wild-type (WT) after intratracheal administration of bleomycin (BLM). This means that non-hematopoietic cells in the fibrotic phase produce an attractant, which causes SatM to migrate to the affected area and cause pulmonary fibrosis.

Thus, non-hematopoietic cells in the fibrotic stage are involved in occurrence of fibrosis, like hematopoietic cells. To identify molecules important in an onset of fibrosis, mRNA and protein expression in lungs of WT mice in the fibrotic phase after intratracheal administration of BLM were investigated. Among differentially expressed mRNAs and proteins, RNA-binding motif protein 7 (RBM7) was significantly increased after BLM treatment, as shown in Fig. Id. Of note, in lung sections of patients with pulmonary fibrosis, expression of RBM7 was significantly elevated in areas of progressive fibrosis as compared to non-fibrotic control (Fig. 1e). Therefore, it was clarified that Rbm7 is involved in pathophysiology of fibrosis onset in humans.

To investigate a pathophysiological role of this protein, Rbm7-/- mice born at Mendelian ratio and normally grown were created. A population of immune cells was also normal in the Rbm7-/- mice, but interestingly, the Rbm7-/- mice were significantly resistant to BLM-induced fibrosis as shown by the survival rate in Fig. If. Moreover, by in vivo magnetic resonance imaging (MRI) analysis, fibrosis in the Rbm7-/- mice was dramatically reduced, as shown in Fig. 1g. The Rbm7-/- mice at the fibrotic stage also showed reduced collagen and α-smooth muscle actin deposition in the lungs as shown in Fig. 1h and lack of accumulation of hydroxyproline (fibrotic marker) as shown in Fig. 1i. Further, as shown in Fig. 1j, BLM-induced expression of profibrotic markers such as collagen 1α (collal) and TGFβ1 (tgfb1) was significantly suppressed in the lungs of Rbm7-/- mice. As shown in Fig. 1k, SatM was not recruited to the lungs of Rbm7-/- mice. Putting these findings together, it is shown that the Rbm7-/- mice are resistant to pulmonary fibrosis.

Furthermore, whether Rbm7 is required for the onset of fibrosis in other tissues was investigated. Figs. 1l to 1o show models of hepatic fibrosis induced by choline-deficient-L-amino acid-high-fat diet (CDA-HFD, n = 3). As shown in Fig. 1l to Fig. 1o, it was confirmed that choline-deficient L-amino acid defined high-fat diet (CDA-HFD) caused fatty liver to the same extent in WT and Rbm7-/- mice, but the hepatic fibrosis induced by CDA-HFD was remarkably suppressed in the Rbm7-/- mice, and RBM7 expression was highly elevated in the fibrotic liver.

Also, Fig. 1p to Fig. 1s show models of renal fibrosis induced by unilateral ureteral obstruction (UUO, n = 3). As shown in Fig. 1p to Fig. 1s, similar findings (renal fibrosis were significantly suppressed in Rbm7-/- mice) were also observed in a mouse renal fibrosis model induced by unilateral ureteral obstruction (UUO). Although not shown, it was confirmed that similar findings were observed in another pulmonary fibrosis model induced by HCl. These findings suggest that Rbm7 is important for the onset of fibrosis in various mouse tissues.

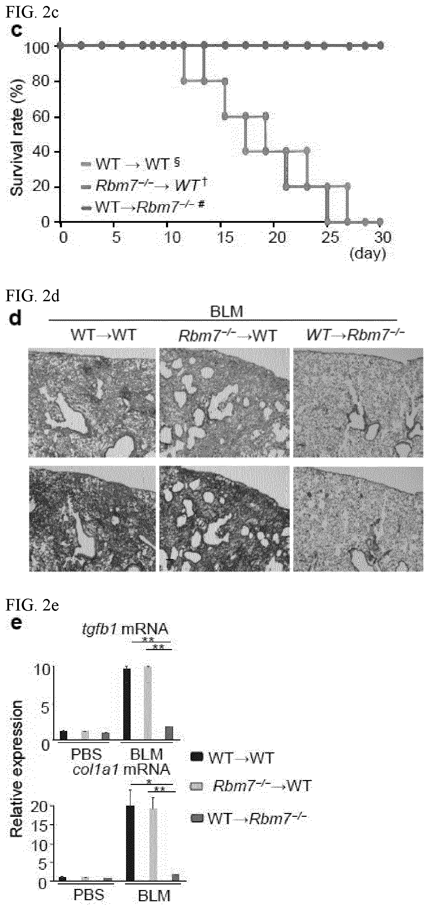

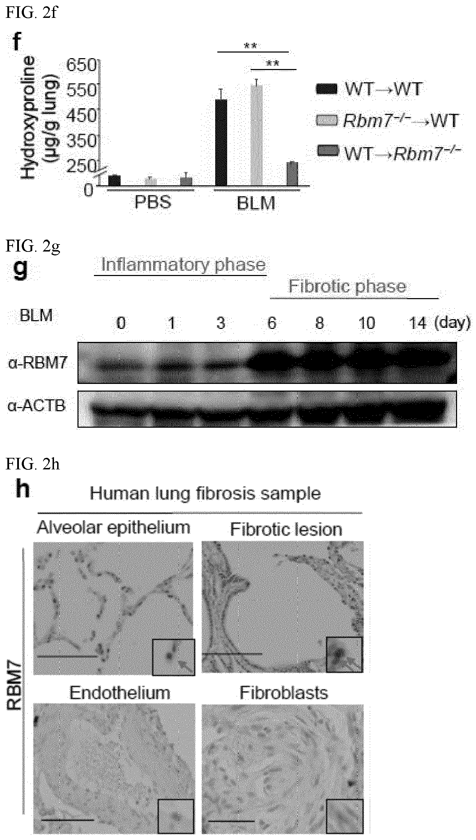

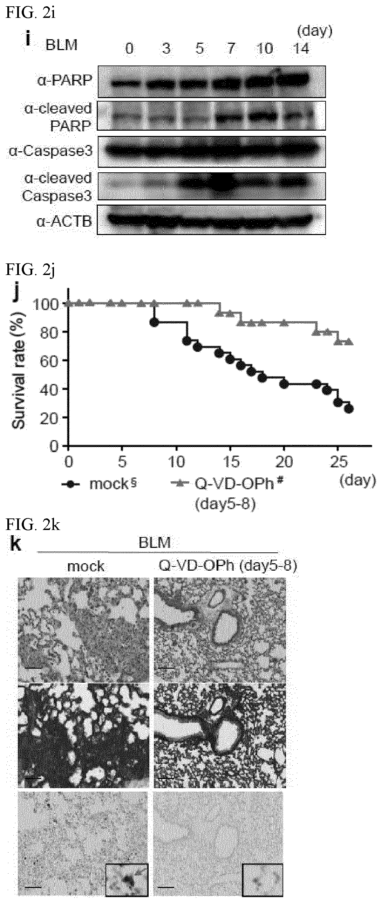

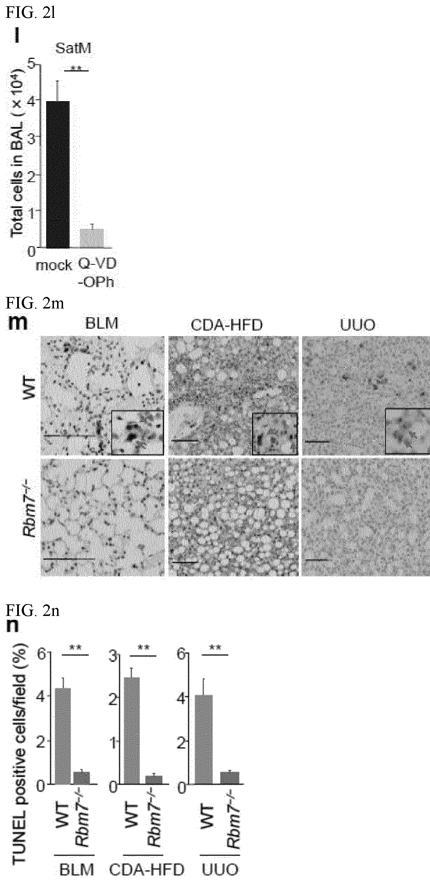

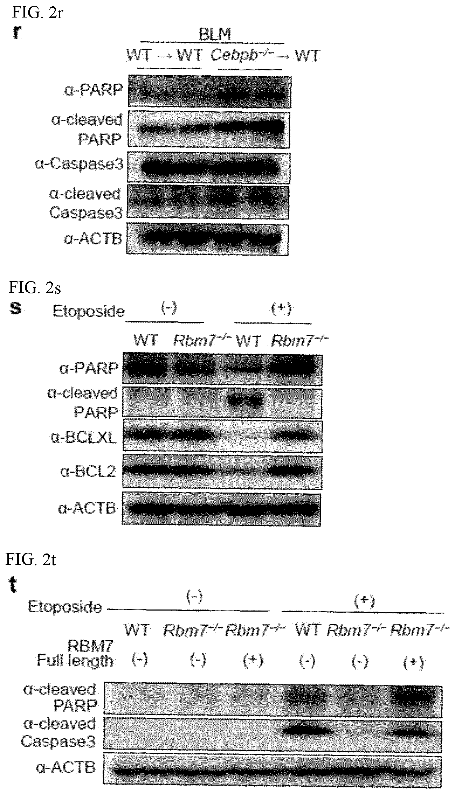

To investigate which cell type is important for BLM-induced fibrosis, RBM7 expression in hematopoietic cells (CD45+) and non-hematopoietic cells (CD45-) was investigated. Fig. 2a to Fig. 2t show that Rbm7-controlled apoptosis plays an important role in the onset of fibrosis. * Indicates P < 0.05, and ** indicates P < 0.01 by Student's t test. Survival curves were analyzed by log rank test. P values between § and † and between # and † were both P < 0.01. All error bars indicate SD. Similar results were also obtained in 5 or more (Fig. 2e to Fig. 2i, Fig. 2s, Fig. 2t) and 3 or more (Fig. 2a to Fig. 2d, Fig. 2j to Fig. 2r) experiments independently performed.

As shown in Fig. 2a and Fig. 2b, it was found that the protein was preferentially expressed in the non-hematopoietic cells. Although not shown, it was confirmed that various macrophages derived from Rbm7-/- mice showed normal response to stimulation. To confirm that RBM7 expression in the non-hematopoietic cells contributes to the onset of fibrosis, WT or Rbm7-/- mice were lethally irradiated and bone marrow (BM) was reconstituted from WT or Rbm7-/- mice, thereby creating chimeric mice. As shown in Fig. 2c to Fig. 2f, in the BLM model, while chimeric mice (Rbm7-/-→WT) in which hematopoietic cells were deficient in Rbm7 expression normally caused pulmonary fibrosis, mice with Rbm7-deficient non-hematopoietic cells (WT-Rbm7-/-) were resistant to fibrosis in a lung model. Although not shown, it was confirmed that similar results were obtained in a liver model and a kidney model. Therefore, RBM7 expressed in non-hematopoietic cells was shown to be essential for the onset of fibrosis.

Next, an underlying mechanism by which Rbm7 loss prevents fibrosis was investigated. As shown in Fig. 2g, RBM7 expression began to rise from a beginning of the fibrotic phase, and this molecule was highly expressed in both mouse and human lung epithelial cells at the onset of fibrosis as shown in Figs. 2b and 2h. Consistent with the timing of RBM7 expression, BLM induced severe and continuous apoptosis, as shown in Fig. 2i. From these findings, a relationship between Rbm7 and apoptosis in the onset of pulmonary fibrosis was investigated. Here, it is known that cell death, regardless of whether it is induced by drug administration, infection or physical trauma, can progress fibrosis by release of endogenous ligands from dead cells. To investigate the role of apoptosis in the fibrotic stage, a widely used anti-apoptotic reagent Q-VD-OPh was administered to mice at the beginning of the fibrotic stage after BLM administration.

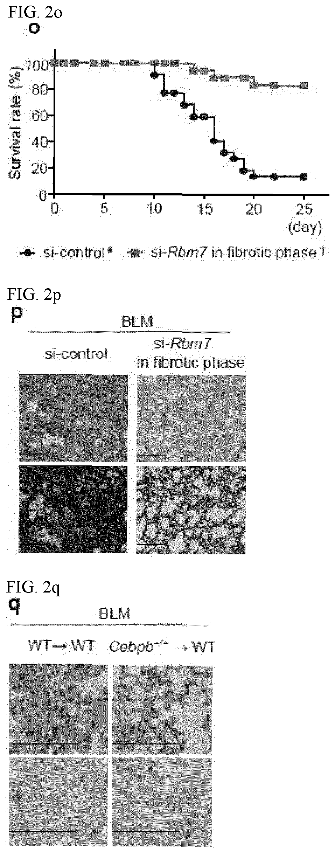

As shown in Fig. 2j to Fig. 2l, fibrosis inhibition was not suppressed by administration of Q-VD-OPh in the inflammatory stage, and was suppressed only by the administration of Q-VD-OPh in the fibrotic phase, and conferred resistance to fibrosis, and SatM migration was significantly suppressed. In fact, as shown in Fig. 2m and Fig. 2n, in the fibrotic stage, a large number of dead cells were detected in the lungs of WT mice, but were not detected in the BLM-treated Rbm7-/- mice. Moreover, as shown in Fig. 2m and Fig. 2n, Rbm7-/- cells derived from liver and renal fibrosis mouse models were also resistant to cell death. Furthermore, to confirm that suppression of Rbm7 in the fibrotic phase confers resistance to fibrosis, siRNA against Rbm7 was administered in vivo to the lungs of WT mice. As shown in Fig. 2o and Fig. 2p, suppression of Rbm7 in the WT mice conferred resistance to fibrosis. In contrast, as shown in Fig. 2q and Fig. 2r, SatM deficient cebpb-/- chimeric mice showed apoptosis comparable to lungs at the beginning of fibrosis.

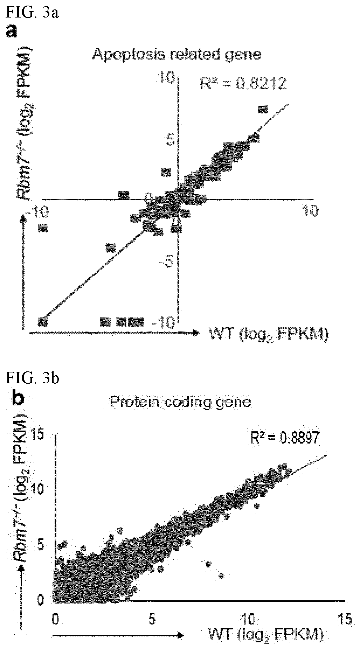

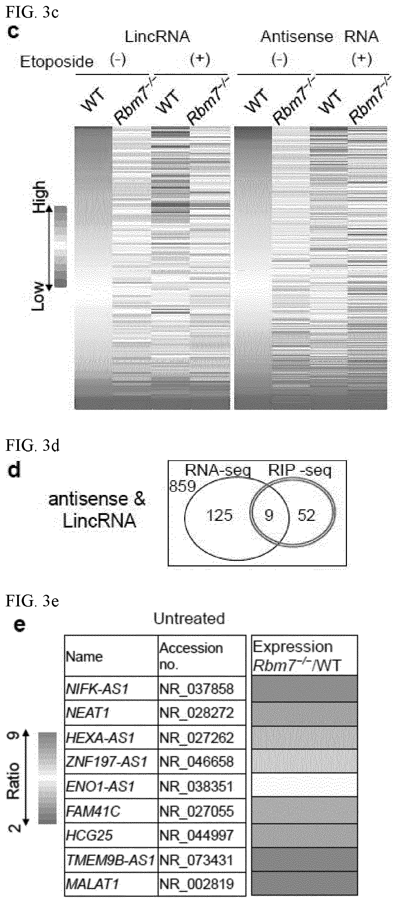

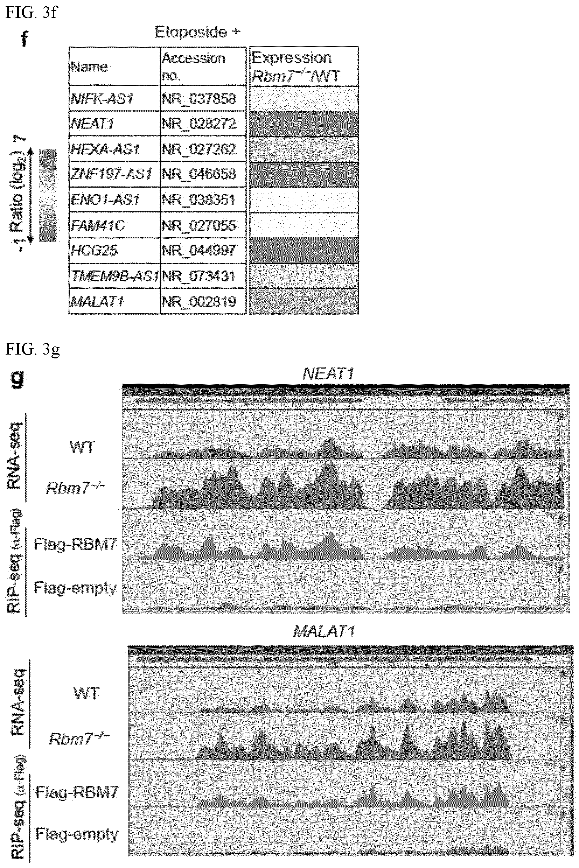

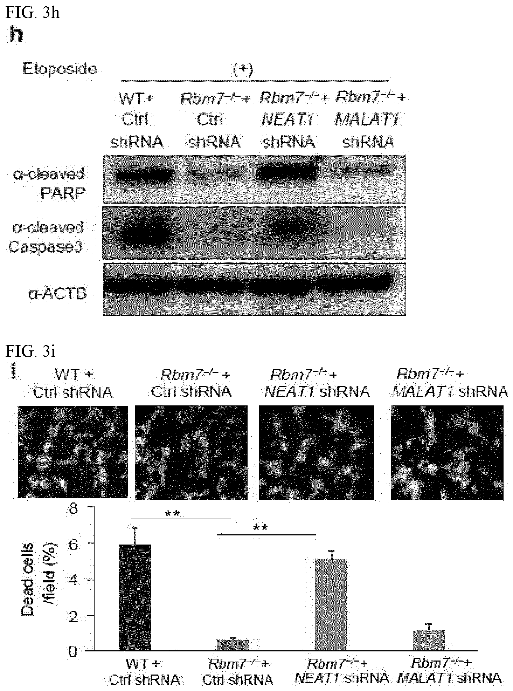

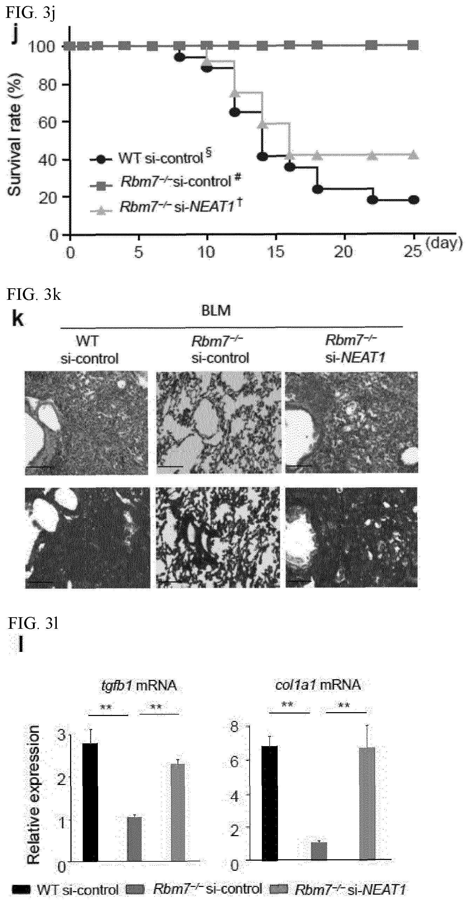

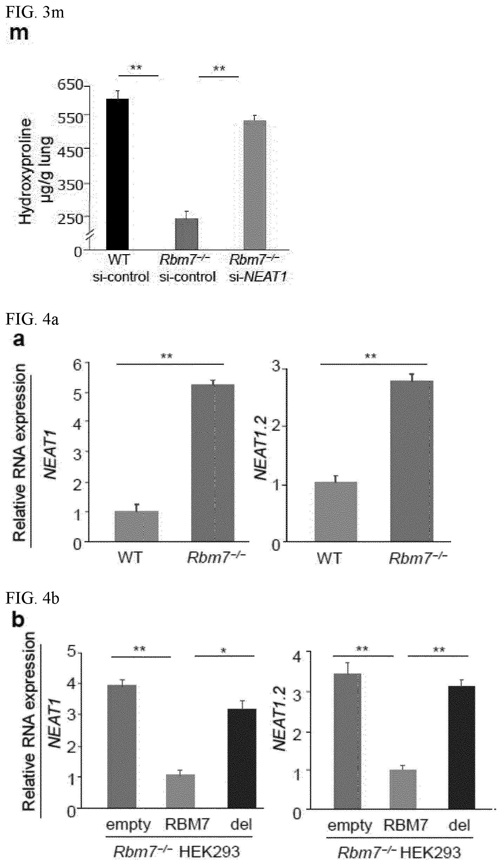

Furthermore, Fig. 3a to Fig. m show regulation of non-coding RNA by RBM7. * Indicates P < 0.05, and ** indicates P < 0.01 by Student's t test. Similar results were also obtained in 5 or more (Fig. 3a, Fig. 3b, Fig. 3g, Fig. 3i) and 3 or more (Fig. 3c to Fig. 3f, Fig. 3h, Figs. 3j to n) experiments independently performed.

It was also confirmed that there was no difference in apoptosis-related pathways between WT and Rbm7-/- HEK293 cells, and there was no difference in gene expression as shown in Fig. 3a. Interestingly, as shown in Fig. 3b and Fig. 3c, expression of long ncRNA (lncRNA) and antisense RNA (not a protein-coding gene) was significantly different between WT and Rbm7-/- cells. Therefore, RNA immunoprecipitation sequencing (RIP-seq) analysis was performed to identify potential targets for IncRNA and antisense RNAs regulated by RBM7. It was confirmed that some IncRNA including NIPK-AS1, MALAT1 and NEAT1 and antisense RNA were precipitated (determined from RIP-seq data) and increased (determined by RNA-seq and qPCR analysis). Furthermore, 9 molecules were confirmed to overlap between the two analyses, as shown in Fig. 3d.

Among these potential targets of RBM7, their expression levels in RNA-seq data with and without etoposide treatment were further investigated, and it was found that ncRNA such as NEAT1 is highly expressed in any situations, as shown in Fig. 3e, Fig. 3f, and Fig. 3g. To investigate whether any of these ncRNAs was associated with resistance to cell death induced by loss of RBM7, an effect of shRNA-mediated knockdown (KD) of NEAT1 and MALAT1 in WT or Rbm7-/- cells was investigated. As shown in Fig. 3h and Fig. 3i, suppression of NEAT1 but not MALAT1 rescued an ability of etoposide to induce cell death in Rbm7-/- HEK293 cells. Furthermore, to confirm that suppression of NEAT1 in the Rbm7-/- mice led to resistance to fibrosis, siRNA against this ncRNA was administered in vivo to the Rbm7-/- mouse lungs. Interestingly, the suppression of NEAT1 in the Rbm7-/- mice resulted in the onset of fibrosis, as shown in Fig. 3j to Fig. 3m. These findings indicated that RBM7 plays an important role in inducing cell death by controlling expression of selected target ncRNAs.

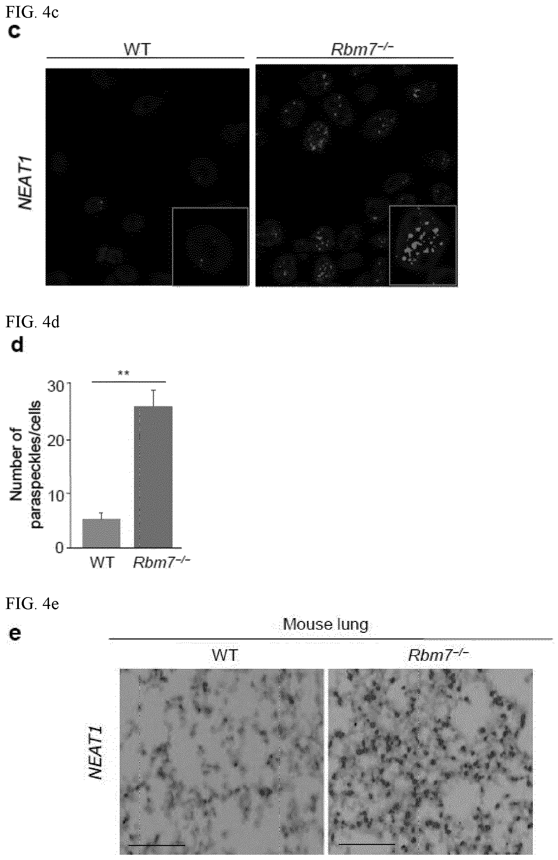

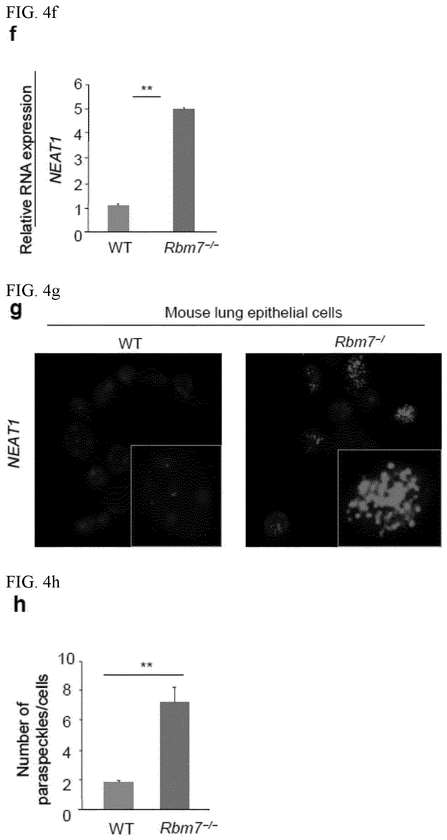

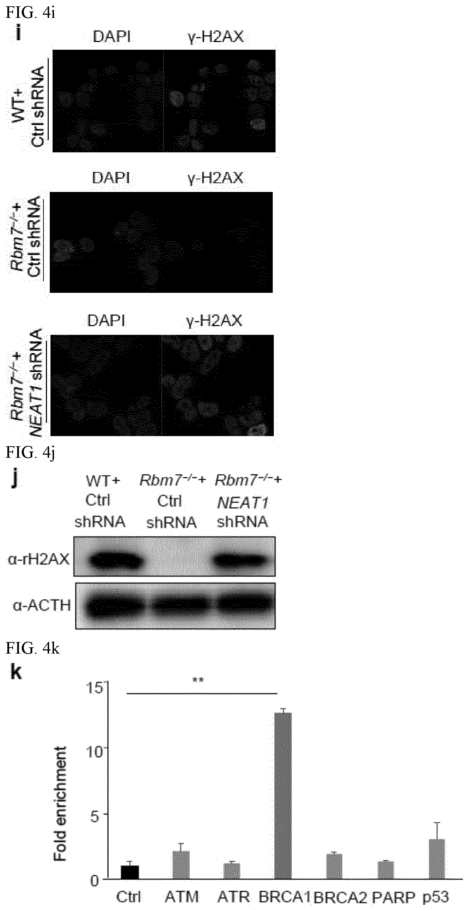

Fig. 4a to Fig. 4m show that BRCA1 localizes to RBM7-regulated NEAT1-containing paraspeckle. * Indicates P < 0.05, and ** indicates P < 0.01 by Student's t test. Survival curves were analyzed by log rank test. Similar results were also obtained in 5 or more (Fig. 4a to Fig. 4d, Fig. 4i, Fig. 4j) and 3 or more (Fig. 4e to Fig. 4h, Fig. 4k to Fig. 4m) experiments independently performed.

Both NEAT1 and NEAT1.2 RNA expressions were elevated in Rbm7-/- HEK293 cells but were reversed by re-expression of full length RBM7, as shown in Fig. 4a and Fig. 4b. The reversal did not occur by re-expression of RBM7 lacking an important RNA binding site. It was also confirmed that NEAT1, MALAT1 and U1 snRNA, but not TERT, were strongly bound to RBM7 by RIP and CLIP-qPCR analysis. RBM7 was partially co-localized with NEAT1. Furthermore, Rbm7 was not involved in transcription of NEAT1. This indicates that RBM7 is involved in decay of NEAT1 ncRNA. Here, a recent report suggests that NEAT1 is an essential component of paraspeckle nucleoli. Therefore, localization of RBM7 and NEAT1 with respect to paraspeckle was investigated. As shown in Fig. 4c to Fig. 4h, consistent with NEAT1 RNA decay being suppressed in RBM7-deficient cells, in both mouse lung and human cell lines, increases in the number of NEAT1-containing paraspeckle under Rbm7 deficiency and frequency of paraspeckle positive cells were confirmed. To investigate whether increased NEAT1 expression was associated with suppression of cell death under RBM7 deficiency, shRNA was used to inhibit NEAT1 in Rbm7-/- cells by lentiviral system. As shown in Fig. 4i and Fig. 4j, suppression of NEAT1 in Rbm7-/- cells caused activation of a DNA damage response associated with cell death.

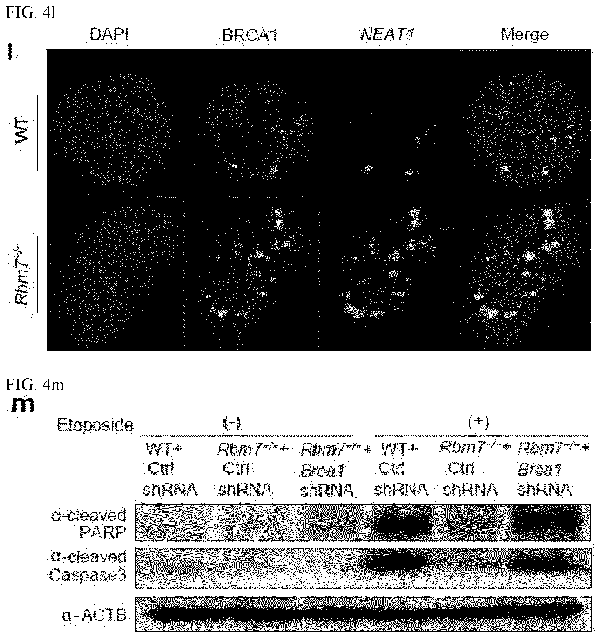

To elucidate factors that link Rbm7 deficiency, cell death suppression, and NEAT1 accumulation, an association between NEAT1 and various proteins involved in cell death was investigated. As shown in Fig. 4k, by RNA-immunoprecipitation-qPCR analysis, it was found that NEAT1 was associated with BRCA1 (which is known to be a key factor for DNA repair proteins), and was not associated with other DNA repair or cell death-related factors. As shown in Fig. 4l, BRCA1 co-localized specifically with NEAT1, and BRCA1 and NEAT 1-containing paraspeckle was more abundant in Rbm7-/- than in WT HEK293 cells. Although not shown, it was also confirmed by CLIP-qPCR analysis that NEAT1 was strongly bound to BRCA1 unlike other ncRNAs such as MALAT1 and TERT. Then, it was also confirmed by RNA antisense purification (RAP) using a NEAT1 probe that BRCA1 was strongly bound to NEAT1 in the same manner as RBM7 and FUS (known paraspeckle markers). Further, it was also confirmed that DNA damage induced BRCA1 dispersion from NEAT1 paraspeckle. Finally, as shown in Fig. 4m, lentivirally shRNA-mediated knockdown of BRCA1 reestablished an ability of Rbm7-/- cells to undergo cell death induction in response to etoposide. These findings indicate that RBM7 regulates cell death by controlling NEAT1 levels and BRCA1 localization in the nucleus.

Recent studies have shown that another mRNA, MORRBID, is involved in cell death and is important for survival of hematopoietic cells such as inflammatory monocytes, neutrophils and eosinophils. MORRBID regulates transcription of pro-apoptotic protein Bim by promoting enrichment of PRC complex in its promoter region. In contrast, RBM7 is expressed mainly in non-hematopoietic cells and does not directly control expression of cell death-related molecules. Therefore, ncRNA-mediated regulation of cell death may be common to many cell types.

Several lung disease diagnostic markers are widely used, including KL-6, SP-D and MCP-125. However, these are indirect markers and few markers are available for detection of progressive fibrosis. In the present invention, RBM7 was found to be highly expressed in a lung area with progressive fibrosis, and the protein RBM7 was found to be strongly associated with occurrence of pulmonary fibrosis, by an expression pattern of Rbm7 in mice, and by imparting resistance to fibrosis by loss of Rbm7. It is also speculated that RBM7 functions similarly in humans. Therefore, RBM7 can be a useful diagnostic marker for fibrosis.

Cell death was significantly suppressed in Rbm7-/- cells, indicating that RBM7 is a novel factor involved in cell death regulation. DNA damage is known to induce release of p53 from a MDM2 complex. Then, p53 is known to induce expression of BH3-only proteins such as Noxa and Puma. These two molecules are associated with anti-apoptotic gene Bcl-2 after inhibition of cell death, and further, p53 is known to be involved in expression of pro-apoptotic gene Bax. Therefore, activation of p53 is important for induction of cell death. Recently, p53 has been reported to induce formation of NEAT1-containing paraspeckle. The present invention has shown that RBM7 regulates NEAT1 levels and the association of BRCA1 with NEAT1 paraspeckle. Thus, RBM7 is considered to be a management system that controls cell death by inducing dissociation of accumulated NEAT1 paraspeckle, thereby preventing repair of irreversibly damaged cells. In the fibrotic stage, RBM7 expression was highly elevated, and an extremely large number of dead cells were detected. As a result, several factors, such as chemoattractants secreted by non-hematopoietic cells leading to death, induced recruitment of prefibrotic monocyte SatM to an affected lesion site. Therefore, it is considered that RBM7 recognizes the occurrence of SatM-induced fibrosis through decay of non-coding RNA and regulation of cell death. Furthermore, Rbm7 may share similar functions in the situation of fibrosis onset between mice and humans, so considering that an inhibitor of RBM7 has been developed, the inhibitor of RBM7 will be an effective medicine effective for fibrosis.

## Claims

1. An antifibrotic agent, comprising a substance that suppresses expression of RNA-binding motif protein 7 (RBM7) as an active ingredient.

2. The antifibrotic agent according to claim 1, wherein the substance is at least one nucleic acid medicine selected from the group consisting of siRNA, shRNA, dsRNA, miRNA and an antisense nucleic acid against RBM7.

3. The antifibrotic agent according to claim 1 or 2, which is used for treatment, prevention or progression prevention of fibrosis.

4. The antifibrotic agent according to claim 3, wherein the fibrosis is fibrosis expressing RBM7.

5. A biomarker of fibrosis, comprising RBM7.

6. The biomarker according to claim 5, wherein the fibrosis is selected from the group consisting of pulmonary fibrosis, hepatic fibrosis, renal fibrosis, scleroderma, cardiac fibrosis, and myelofibrosis.

7. A method for detecting fibrosis, comprising a step of performing a detection of RBM7 in a biological sample derived from a test subject.

8. The method according to claim 7, wherein the biological sample is a blood sample.

9. The method according to claim 7 or 8, wherein the detection is performed using an anti-RBM7 antibody.

10. A diagnostic agent for fibrosis, comprising an anti-RBM7 antibody.

11. A method for screening an active ingredient of an antifibrotic agent, comprising the following steps (A) to (C):
(A) bringing a test substance into contact with a cell expressing RBM7,
(B) measuring an expression level of RBM7 in the cell, and
(C) selecting the test substance as a candidate for an active ingredient of an antifibrotic agent when ability to suppress expression of RBM7 is recognized.
